# EUROPÄISCHE PATENTSCHRIFT

(11) **EP 1 599 203 B1**
(45) Veröffentlichungstag und Bekanntmachung des Hinweises auf die Patenterteilung: **23.10.2013**
(21) Anmeldenummer: 04712489.6
(22) Anmeldetag: 19.02.2004
(51) Int. Cl.: A61K 31/422, A61P 3/00, C07D 413/12, C07D 419/12, C07D 471/04

(54) **1,3-SUBSTITUIERTE CYCLOALKYLDERIVATE MIT SAUREN, MEIST HETEROCYCLISCHEN GRUPPEN; VERFAHREN ZU IHRER HERSTELLUNG UND IHRE VERWENDUNG ALS ARZNEIMITTEL**
1,3-SUBSTITUTED CYCLOALKYL DERIVATIVES CONTAINING ACIDIC, MAINLY HETEROCYCLIC GROUPS, CORRESPONDING PRODUCTION METHOD AND USE OF SAID DERIVATIVES AS MEDICAMENTS
DÉRIVÉS DE CYCLOALKYLE 1,3-SUBSTITUÉS CONTENANT DES GROUPES ACIDES PRINCIPALEMENT HÉTÉROCYCLIQUES, PROCÉDÉ DE PRÉPARATION ASSOCIÉ ET UTILISATION DE CES DÉRIVÉS COMME MÉDICAMENTS

(30) Priorität: 27.02.2003 DE 10308351
(43) Veröffentlichungstag der Anmeldung: 30.11.2005
(73) Patentinhaber: Sanofi-Aventis Deutschland GmbH, 65929 Frankfurt am Main (DE)
(72) Erfinder: GOERLITZER, Jochen, 60594 Frankfurt am Main (DE); GLOMBIK, Heiner, 65719 Hofheim (DE); FALK, Eugen, 60529 Frankfurt (DE); GRETZKE, Dirk, 60596 Frankfurt (DE); KEIL, Stefanie, 65719 Hofheim (DE); SCHAEFER, Hans-Ludwig, 65239 Hochheim (DE); STAPPER, Christian, 55118 Mainz (DE); WENDLER, Wolfgang, 65618 Selters (DE)
(86) Internationale Anmeldenummer: PCT/EP2004/001582
(87) Internationale Veröffentlichungsnummer: WO 2004/075891

(56) Entgegenhaltungen:
- WO-A-00/64876
- WO-A-01/21602
- WO-A-02/30895
- WO-A-02/100403
- WO-A-03/043985

## Beschreibung

Die Erfindung betrifft 1,3- substituierte Gycloalkylderivate mit sauren, meist heterocyclischen Gruppen sowie deren physiologisch verträgliche Salze und physiologisch funktionelle Derivate.

Es sind bereits strukturähnliche Verbindungen zur Behandlung von Hyperlipidämie und Diabetes im Stand der Technik beschrieben (WO 2000/64876).

Der Erfindung lag die Aufgabe zu Grunde, Verbindungen zur Verfügung zu stellen, die eine therapeutisch verwertbare Modulation des Lipid- und/oder Kohlehydratstoffwechsels erlauben und sich somit zur Prävention und/oder Behandlung von Krankheiten wie Typ 2 Diabetes und Atherosklerose sowie deren vielfältigen Folgeerkrankungen eignen.

Überraschenderweise wurde eine Serie von Verbindungen gefunden, die die Aktivität von PPAR Rezeptoren modulieren. Insbesondere eignen sich die Verbindungen zur Aktivierung von PPARalpha und PPARgamma, wobei das Ausmaß der relativen Aktivierung je nach Verbindungen unterschiedlich sein kann.

Die Erfindung betrifft daher Verbindungen der Formel I 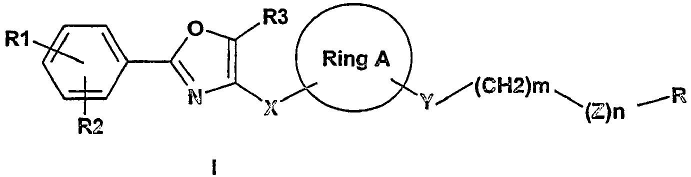 worin bedeuten:
- Ring A: Cyclohexan-1,3-diyl;
- R1, R2: unabhängig voneinander H, F, Br, Cl, SF₅, S-(C₁-C₆)-Alkyl, CF₃, OCF₃, (C₁-C₆)-Alkyl, O-(C₁-C₆)-Alkyl, SCF₃, Phenoxy, OCF₂CHF₂, OCF₂CF₃, (C₁-C₆)-Alkyl-(C₁-C₆)-Alkoxy, O(C₁-C₆)-Alkyl-(C₁-C₆)-Alkoxy, Benzyloxy;
- R3: H, CF₃, (C₁-C₆)-Alkyl, (C₃-C₈)-Cycloalkyl, Phenyl;
- X: (C₁-C₆)-Alkandiyl, wobei in der Alkandiylgruppe ein oder mehrere Kohlenstoffatome durch Sauerstoffatome ersetzt sein können;
- Y: S, O, Bindung;
- m: 1 bis 3;
- n: 0 oder 1;
- Z: O, S, CO oder CO-NH;
- R: H, OH, CH₂-CO-NH-OH, CH₂-CO-NH-(C₁-C₆)-Alkyl, CH₂-CO-NH-(C₁-C₆)-Alkoxy, NR4R5 oder ein 5 - 12 gliedriger mono- oder bicyclischer, ungesättigter, teilweise ungesättigter oder gesättigter Ring, der ein bis vier Heteroatome aus der Gruppe N, O oder S enthalten und benzanneliert sein kann, wobei der 5-12 gliedrige Ring weitere Substituenten wie F,Cl, Br, CN, SH, COOH, (C₁C₄)-Alkyl, (C₁-C₆)-Alkoxy, SO₂-(C₁-C₄)-Alkyl, NO₂, CF₃, OCF₃, (C₁-C₆)-Alkyl-(C₁-C₆)-Alkoxy, (C₁-C₆)-Alkoxy-(C₁-C₆)-Alkoxy, (C₁-C₆)-Alkoxy-Phenyl, Phenoxy, NHSO₂CF₃ oder B(OH)₂ tragen kann;
- R4: H, (C₁-C₆)-Alkyl;
- R5: OH, NH₂, SO₂-CF₃, SO₂-Phenyl-CF₃, CO-CF₃, (C₁-C₆)-Alkoxy, Phenyl, das gegebenenfalls substituiert sein kann durch CH₃ und COOH;
- R4 und R5: zusammen mit dem sie tragenden N-Atom einen 5 gliedrigen aromatischen Heterocyclus bilden, der gegebenenfalls wiederum an einen aromatischen 5 - 7 gliedrigen Ring mit gegebenenfalls ein bis vier N-Atomen anneliert ist und substituiert sein kann durch: F, Cl, Br, CF₃, OCF₃, COOH, SO₂CH₃, CN, (C₁-C₄)-Alkoxy, (C₁-C₄)-Alkyl, (C₁-C₆)-Alkyl-Phenyl, (C₁-C₆)-Alkyl-(C₁-C₆)-Alkoxy, (C₁-C₆)-Alkoxy-(C₁-C₆)-Alkoxy, (C₁-C₆)-Alkoxy-Phenyl, Phenoxy;
sowie deren physiologisch verträgliche Salze, wobei die Verbindungen der Formel I ausgenommen sind mit Y und Z = O und R = Phenyl-COOH oder Phenyl-(C₁-C₄)-Alkyl und solche mit Y = O, n = 0 und R = Phenyl-COOH oder Phenyl-(C₁-C₄)-Alkyl.

Bevorzugt sind Verbindungen der Formel I, worin bedeuten
- Ring A: Cyclohexan-1,3-diyl; und
- X: (C₁-C₆)-Alkandiyl, worin ein Kohlenstoffatom durch ein Sauerstoffatom ersetzt sein kann;
oder Verbindungen der Formel I, worin bedeuten
- Ring A: Cyclohexan-1,3-diyl und
- X: CH₂-O;
oder Verbindungen der Formel I, worin bedeuten
- Ring A: Cyclohexan-1,3-diyl;
- X: CH₂-O;
- Y: O.

Besonders bevorzugt sind Verbindungen der Formel I, worin der zentrale Cycloalkan-1,3-diylring cis verknüpft ist
oder Verbindungen der Formel I, worin bedeuten
- R1/R2: H, (C₁-C₄)-Alkyl, (C₁-C₄)-Alkoxy;
- R3: (C₁-C₄)-Alkyl.

Besonders bevorzugt sind ferner Verbindungen der Formel I, worin bedeuten
- Y: O;
- m: 3;
- n: 0;
oder Verbindungen der Formel I, worin bedeuten
- Y: O;
- m: 2;
- n: 0;
oder Verbindungen der Formel I, worin bedeuten
- Y: O;
- m: 2;
- n: 1;
- Z: O;
oder Verbindungen der Formel I, worin bedeuten
- Y: O;
- m: 1;
- n: 0;
oder Verbindungen der Formel I, worin bedeuten
- Y: Bindung;
- m: 1;
- n: 0;
der Verbindungen der Formel I, worin bedeuten
- Y: Bindung;
- m: 1;
- n: 1;
- Z: O.

Ganz besonders bevorzugt sind Verbindungen der Formel I, worin bedeuten
- Y: O;
- m: 3;
- n: 0;
- R: Tetrazol, NHSO₂CF₃;
oder Verbindungen der Formel I, worin bedeuten
- Y: O;
- m: 2;
- n: 0;
- R: Tetrazol, NHSO₂CF₃ oder NR4R5, das für Indol oder 6-Azaindol steht und substituiert sein kann durch F, Br, CN, COOH, (C₁C₄)-Alkyl, (C₁-C₄)-Alkoxy, SO₂-CH₃, (C₁-C₆)-Alkoxy-(C₁-C₆)-Alkoxy oder Benzoxy;
oder Verbindungen der Formel I, worin bedeuten
- Y: O;
- m: 2;

- n: 1;
- Z: O;
- R: Phenyl oder Thiophen, die weitere Substituenten wie F, COOH, (C₁-C₄)-Alkyl, (C₁-C₄)-Alkoxy, NO₂, CF₃, Benzyloxy oder B(OH)₂ tragen können;
oder Verbindungen der Formel I, worin bedeuten:
- Y: O;
- m: 1;
- n: 0;
- R: Phenyl-NHSO₂CF₃, Phenyl-B(OH)₂,
oder Verbindungen der Formel I, worin bedeuten
- Y: Bindung;
- m: 1;
- n: 0;
- R: NR4R5, das für Pyrrol oder Indol steht und durch COOH substituiert ist;
oder Verbindungen der Formel I, worin bedeuten
- Y: Bindung;
- m: 1;
- n: 1;
- Z: O;
- R: Thiophen oder Benzothiophen, die substituiert sein können durch COOH, Cl, CF₃.

Die vorliegende Erfindung umfasst ebenfalls alle Kombinationen der "bevorzugten Ausführungsformen" der hierin beschriebenen Erfindung.

Die Alkyl-Reste in den Substituenten R, R1, R2, R3, R4 und R5 können sowohl geradkettig wie verzweigt sein.

Unter Aryl wird ein aromatisches carbocyclisches mono- oder bicyclisches RingSystem verstanden, das 6 bis 10 Atome im Ring oder in den Ringen enthält.

Heteroaryl ist ein mono- oder bicyclisches aromatisches Ringsystem mit 4 bis 11 Ringgliedern, worin mindestens ein Atom im Ringsystem ist ein Heteroatom aus der Reihe N, O und S.

Die Verbindungen der Formel I enthalten mindestens zwei Assymmetriezentren und können darüber hinaus mehr enthalten. Daher können die Verbindungen der Formel I in Form ihrer Racemate, racemischen Mischungen, reinen Enantiomere, - Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Al-kalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

### Verwendung

Diese Erfindung bezieht sich weiterhin auf die Verwendung von Verbindungen der Formel I und ihren pharmazeutischen Zusammensetzungen als PPAR-Rezeptor-Liganden. Die erfindungsgemäßen PPAR-Rezeptor-Liganden eignen sich als Modulatoren der Aktivität der PPAR Rezeptoren. Peroxisomen-Proliferatoren-Aktivierte Rezeptoren (PPAR) sind durch Liganden aktivierbare Transkriptionsfaktoren, die zur Klasse der Kern-Hormon-Rezeptoren gehören. Es existieren drei PPAR Isoformen, PPARalpha, PPARgamma und PPARdelta, die von verschiedenen Genen kodiert werden (Peroxisome proliferatoractivated receptor (PPAR): structure, mechanisms of activation and diverse functions: Motojima K, Cell Struct Funct. 1993 Oct; 18(5): 267-77).

Von PPARgamma existieren zwei Varianten, PPARgamma₁ und gamma₂, die das Ergebnis eines alternativen Einsatzes von Promotoren und einer differenziellen mRNA-Spleißung (Vidal-Puig et al. J. Clin. Invest., 97:2553-2561, 1996) sind. Die verschiedenen PPAR Rezeptoren besitzen eine unterschiedliche Gewebsverteilung und modulieren unterschiedliche physiologische Funktionen. Die PPAR-Rezeptoren spielen eine Schlüsselrolle bei unterschiedlichen Aspekten der Regulation einer Vielzahl von Genen, deren Genprodukte direkt oder indirekt am Lipid- und Kohlehydratstoffwechsel entscheidend beteiligt sind. So spielen z. B. PPARalpha Rezeptoren bei der Regulation des Fettsäurekatabolismus oder des Lipoproteinstoffwechsels in der Leber eine wichtige Rolle, während PPARgamma zum Beispiel bei der Regulation der Fettzelldifferenzierung entscheidend beteiligt ist. Darüberhinaus sind PPAR Rezeptoren aber auch an der Regulation vieler weiterer physiologischer Prozesse, auch solcher die nicht direkt mit dem Kohlehydrat- oder Lipidstoffwechsel in Verbindung stehen, beteiligt. Die Aktivität der unterschiedlichen PPAR Rezeptoren kann durch verschiedene Fettsäuren, Fettsäurederivate sowie synthetische Verbindungen in unterschiedlichem Ausmaß moduliert werden. Entsprechende Reviews über Funktionen, physiologische Wirkung und Pathophysiologie siehe: Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63.

Die vorliegende Erfindung betrifft Verbindungen der Formel I, die sich zur Modulierung der Aktivität von PPAR Rezeptoren eignen, insbesondere der Aktivität von PPARalpha und PPARgamma. Je nach Profil der Modulation sind die Verbindungen der Formel I zur Behandlung, Kontrolle und Prohylaxe nachfolgend beschriebener Indikationen, sowie einer Reihe anderer, damit verbundener pharmazeutischer Anwendungen geeignet (siehe z.B. Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63; Jean-Charles Fruchart, Bart Staels and Patrick Duriez: PPARS, Metabolic Disease and Arteriosclerosis, Pharmacological Research, Vol. 44, No. 5, 345-52, 2001; Sander Kersten, Beatrice Desvergne & Walter Wahli: Roles of PPARs in health and disease, NATURE, VOL 405 ,25 MAY 2000, 421-4; Ines Pineda Torra, Giulia Chinetti, Caroline Duval, Jean-Charles Fruchart and Bart Staels: Peroxisome proliferator-activated receptors: from transcriptional control to clinical practice, Curr Opin Lipidol 12: 2001 , 245-254).

Besonders geeignet sind solche Verbindungen zur Behandlung und/oder Prävention von
1.
   - Störungen des Fettsäurestoffwechsels und Glucoseverwertungsstörungen
   - Störungen, bei denen Insulin Resistenz eine Rolle spielt
2. Diabetes mellitus, insbesondere Typ 2 Diabetes einschließlich der Verhinderung der damit verbundenen Folgeerkrankungen. Besondere Aspekte sind dabei die
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden

Organe, des Genitaltraktes, Prostata-Karzinome etc.
- akute und chronische myeloprolifeative Erkrankungen und Lymphome
- Angiogenese
- Neurodegenerative Erkrankungen
- Alzheimersche Krankheit
- Multiple Sklerose
- Morbus Parkinson
- Erythemato-squamöse Dermatosen, wie z.B. Psoriasis (Schuppenflechte)
- Akne vulgaris
- Andere Hautkrankheiten und dermatologische Zustände, die durch PPAR moduliert werden
- Ekzeme und Neurodermitis
- Dermatitiden, wie z.B. seborrhoische Dermatitis oder Lichtdermatitis
- Keratitis und Keratosen, wie z.B. seborrhoische Keratosen, senile Keratosen, aktinische Keratose, photo-induzierte Keratosen oder Keratosis follicularis
- Keloide und Keloid-Prophylaxe
- Warzen, einschließlich Kondylomata oder Kondylomata acuminata
- Human papilloma viral (HPV) Infektionen, wie z.B. venerische Papillomata, virale Warzen, wie z.B. Molluscum contagiosum, Leukoplakie
- Papulöse Dermatosen, wie z.B. Lichen planus
- Hautkrebs, wie z.B. Basalzellkarzinome, Melanome oder kutane T-Zell Lymphome
- Lokalisierte, benigne epidermale Tumore, wie z.B. Keratoderma, epidermale Naevi
- Frostbeulen
- Hoher Blutdruck
- Syndrom X
- Syndrom der polyzystischen Ovarien (PCOS)
- Asthma
- Osteoarthritis
- Lupus erythematodes (LE) oder entzündliche rheumatische Erkrankungen, wie z.B. Rheumatoide Arthritis
- Vaskulitis
- Auszehrung (Kachexie)
- Gicht
- Ischämie/Reperfusions Syndrom
- Akutes respiratorisches Distress Syndrom (ARDS) ("Schocklunge")

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg bis50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind; Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.
Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem *α*-Glukosidase-Inhibitor, wie z.B. Miglitol oder Acarbose, verabreicht.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, *β*3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-*β-*Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.
Bei einer Ausführungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen wurde wie folgt getestet:

### Bestimmung von EC50-Werten von PPAR-Agonisten im zellulären PPARalpha - Test

### Prinzip

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als PPARalpha-Reporterzellinie bezeichnet wird. Sie enthält zwei genetische Elemente, ein Luziferase-Reporterelement (pdeltaM-GAL4-Luc-Zeo) und ein PPARalpha-Fusionsprotein (GR-GAL4-humanPPARalpha-LBD), welches die Expression des Luziferase-Reporterelementes in Abhängigkeit eines PPARalpha-Liganden vermittelt. Das stabil und konstitutiv exprimierte Fusionsprotein GR-GAL4-humanPPARalpha-LBD bindet im Zellkern der PPARalpha-Reporterzellinie über den GAL4-Proteinanteil an die GAL4-DNA-Bindungsmotife 5'-oberhalb des Luziferase-Reporterelementes, das im Genom der Zellinie integriert ist. Ohne Zugabe eines PPARalpha-Liganden ist die Expression des Luziferase-Reportergens nur gering, sofern im Test fettsäuredepletiertes fötales Kälberserum (cs-FKS) verwendet wird. PPARalpha-Liganden binden und aktivieren das PPARalpha-Fusionsprotein und bewirken darüber die Expression des Luciferasereportergens. Die gebildete Luziferase lässt sich über ein entsprechendes Substrat mittels Chemilumineszenz nachweisen.

### Konstruktion der Zelllinie

Die PPARalpha-Reporterzellinie wurde in 2 Stufen hergestellt: Zuerst wurde das Luziferase-Reporterelement konstruiert und stabil in HEK-Zellen transfiziert. Dazu wurden fünf Bindungsstellen des Hefe-Transkriptionsfaktors GAL4 (jeweils 5'-CGGAGTACTGTCCTCCGAG-3') 5'-oberhalb eines 68 bp langen minimalen MMTV-Promotors (Genbank-Accession # V01175) einkloniert. Der minimale MMTV-Promotorabschnitt enthält eine CCAAT-Box und ein TATA-Element, um eine effiziente Transkription durch die RNA-Polymerase II zu ermöglichen. Die Klonierung und Sequenzierung des GAL4-MMTV-Konstruktes erfolgte analog wie bei Sambrook J. et. al. beschrieben (Molecular cloning, Cold Spring Harbor Laboratory Press, 1989). Danach wurde 3'-unterhalb des GAL4-MMTV-Elementes das gesamte Luziferasegen von Photinus pyralis (Genbank-Accession # M15077) einkloniert. Nach der Sequenzierung wurde das aus fünf GAL4-Bindungsstellen, MMTV-Promotor und Luziferasegen bestehende Luziferase-Reporterelement in ein Zeocin-Resistenz vermittelndes Plasmid umkloniert, um zu dem Plasmid pdeltaM-GAL4-Luc-Zeo zu gelangen. Dieser Vektor wurde nach den Angaben in Ausubel, F.M. et al. (Current protocols in molecular biology, Vol. 1-3, John Wiley & Sons, Inc., 1995) in HEK-Zellen transfiziert. Danach wurde unter Verwendung von zeocinhaltigem Medium (0,5 mg/ml) ein geeigneter stabiler Zellklon selektioniert, der eine möglichst niedrige Basalexpression des Luziferasegens zeigte.

In einem zweiten Schritt wurde das PPARalpha-Fusionsprotein (GR-GAL4-humanPPARalpha-LBD in den beschriebenen stabilen Zellklon eingebracht. Dazu wurde zunächst die für die N-terminalen 76 Aminosäuren des Glukocorticoid-Rezeptors (Genbank-Accession # P04150) kodierende cDNA mit dem für die Aminosäuren 1-147 des Hefetranskriptionsfaktors GAL4 (Genbank-Accession # P04386) kodierenden cDNA-Abschnitt verknüpft. Am 3'-Ende dieses GR-GAL4-Konstruktes wurde die cDNA der Ligandenbindungsdomäne des humanen PPARalpha-Rezeptors einkloniert (Aminosäuren S167-Y468; Genbank-Accession # S74349). Das so hergestellte Fusionskonstrukt (GR-GAL4-humanPPARalpha-LBD) wurde in das Plasmid pcDNA3 (Firma Invitrogen) umkloniert, um darin eine konstitutive Expression durch den Cytomegalovirus-Promotor zu ermöglichen. Dieses Plasmid wurde mit einer Restriktionsendonuklease linearisiert und stabil in den bereits beschriebenen, das Luziferase-Reporterelement enthaltenden Zellklon transfiziert. Durch Selektion mit Zeocin (0,5 mg/ml) und G418 (0,5 mg/ml) wurde die fertige PPARalpha-Reporterzellinie isoliert, die ein Luziferase-Reporterelement enthält und konstitutiv das PPARalpha-Fusionsprotein (GR-GAL4-humanPPARalpha-LBD) exprimiert.

### Durchführung des Tests

Die Aktivität von PPARalpha-Agonisten wird in einem 3-Tagestest bestimmt, der nachfolgend beschrieben ist:

### Tag 1

Die PPARalpha-Reporterzellinie wird bis zu einer 80 %-igen Konfluenz in DMEM-Medium (# 41965-039, Invitrogen) kultiviert, das mit folgenden Zusätzen versetzt ist: 10% cs-FKS (fötales Kälberserum; #SH-30068.03, Hyclone), 0,5 mg/ml Zeocin (#R250-01, Invitrogen), 0,5 mg/ml G418 (#10131-027, Invitrogen), 1% Penicillin-Streptomycin-Lösung (#15140-122, Invitrogen) und 2 mM L-Glutamin (#25030-024, Invitrogen). Die Kultivierung erfolgt in Standard-Zellkulturflaschen (# 353112, Becton Dickinson) in einem Zellkulturbrutschrank bei 37°C in Anwesenheit von 5% CO₂. Die zu 80% konfluenten Zellen werden einmal mit 15 ml PBS gewaschen (#14190-094, Invitrogen), mit 3 ml Trypsinlösung (#25300-054, Invitrogen) für 2 min bei 37°C behandelt, in 5 ml des beschriebenen DMEM-Mediums aufgenommen und in einem Zellzählgerät gezählt. Nach der Verdünnung auf 500.000 Zellen/ml werden jeweils 35.000 Zellen pro well einer 96 well-Mikrotiterplatte mit klarem Plastikboden (#3610, Corning Costar) ausgesät. Die Platten werden für 24 h in einem Zellkulturbrutschrank bei 37°C und 5% CO₂ inkubiert.

### Tag 2

Zu testende PPARalpha-Agonisten werden in einer Konzentration von 10 mM in DMSO gelöst. Diese Stocklösung wird in DMEM-Medium (#41965-039, Invitrogen) verdünnt, das mit 5 % cs-FKS (#SH-30068.03, Hyclone), 2 mM L-Glutamin (#25030-024, Invitrogen) und den bereits beschriebenen Antibiotika (Zeocin, G418, Penicillin und Streptomycin) versetzt ist.

Testsubstanzen werden in 11 verschiedenen Konzentrationen im Bereich von 10 µM bis 100 pM getestet. Potentere Verbindungen werden in Konzentrationsbereichen von 1 µM bis 10 pM oder zwischen 100 nM und 1 pM geprüft.

Das Medium der an Tag 1 ausgesäten PPARalpha-Reporterzelllinie wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden. Jede Platte wurde mit einem Standard PPARalpha-Agonisten belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37 °C und 5 % CO₂ inkubiert.

### Tag 3

Die mit den Testsubstanzen behandelten PPARalpha-Reporterzellen werden aus dem Brutschrank entnommen und das Medium abgesaugt. Zur Lyse der Zellen werden 50 µl Bright Glo Reagens (Firma Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Messzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von PPAR-Agonisten werden mit dem Program XL.Fit nach Vorgabe des Herstellers (Firma IDBS) berechnet.

Die PPARalpha-EC50-Werte für die Verbindungen der Beispiele 1 bis 31 liegen in diesem Assay im Bereich von 0,1 nM bis >10 µM.

Die Ergebnisse für die Aktivität einiger erfindungsgemäßer Verbindungen der Formel I sind in der folgenden Tabelle I angegeben:

**Tabelle I**

| **Beispiel Nr.** | **EC50 PPARalpha [nM]** |
|---|---|
| II | 331 |
| VII | 469 |
| XI | 361 |
| XIV | 106 |
| XV | 519 |
| XXIII | 222 |
| XXVI | 24 |

Aus der Tabelle I ist ersichtlich, dass die erfindungsgemäßen Verbindungen der Formel I den PPARalpha-Rezeptor aktivieren und damit zum Beispiel analog zu klinisch verwendeten Fibraten im Organismus eine Triglyceridsenkung bewirken (siehe z.B. J.-Ch. Fruchard et al.,: PPARS, Metabolic Disease and Atherosclerosis, Pharmacological Research, Vol. 44, No. 5, 345-52, 2001; S. Kersten et al.: Roles of PPARs in health and disease, NATURE, VOL 405 ,25 MAY 2000, 421-4;I. Pineda et al.: Peroxisome proliferator-activated receptors: from transcriptional control to clinical practice,Curr Opin Lipidol 12: 2001, 245-254).

### Bestimmung von EC50-Werten von PPAR-Agonisten im zellulären PPARgamma - Test

### Prinzip

Zur Bestimmung der zellulären PPARgamma Aktivität von PPAR-Agonisten wird ein transientes Transfektionssystem eingesetzt. Es basiert auf der Verwendung eines Luziferase-Reporterplasmides (pGL3basic-5xGAL4-TK) und eines PPARgamma-Expressionsplasmides (pcDNA3-GAL4-humanPPARgammaLBD). Beide Plasmide werden vorübergehend (= transient) in humane embryonale Nierenzellen (HEK-Zellen) transfiziert. In diesen Zellen wird dann das Fusionsprotein GAL4-humanPPARgammaLBD exprimiert, das an die GAL4-Bindungsstellen des Reporterplasmides bindet. In Anwesenheit eines PPARgamma-aktiven Liganden wird durch das aktivierte Fusionsprotein GAL4-humanPPARgammaLBD die Expression des Luziferase-Reportergens induziert, was sich nach Zugabe eines Luziferasesubtrates in Form eines Chemilumineszenzsignals nachweisen lässt. Im Unterschied zur stabil transfizierten PPARalpha-Reporterzellinie werden beim zellulären PPARgamma-Test die beiden Komponenten (Luziferase-Reporterplasmid und PPARgamma-Expressionsplasmid) transient in HEK-Zellen transfiziert, weil die stabile und permanente Expression des PPARgamma-Fusionsproteins zelltoxisch ist.

### Konstruktion der Plasmide

Das Luziferase-Reporterplasmid pGL3basic-5xGAL4-TK basiert auf dem Vektor pGL3basic der Firma Promega. Zur Herstellung des Reporterplasmides wurden fünf Bindungsstellen des Hefe-Transkriptionsfaktors GAL4 (jede Bindungsstelle mit der Sequenz 5'-CTCGGAGGACAGTACTCCG-3'), 5'-oberhalb zusammen mit einem 160 bp langen Thymidinkinase-Promotorabschnitt (Genbank-Accession # AF027128) in pGL3basic einkloniert. 3'-unterhalb des Thymidinkinasepromotors liegt das gesamte Luziferasegen von Photinus pyralis (Genbank-Accession # M15077), welches bereits Bestandteil des verwendeten Plasmides pGL3basic ist. Die Klonierung und Sequenzierung des Reporterplasmides pGL3basic-5xGAL4-TK erfolgte analog wie bei Sambrook J. et. al. beschrieben (Molecular cloning, Cold Spring Harbor Laboratory Press, 1989).

Zur Herstellung des PPARgamma-Expressionsplasmides pcDNA3-GAL4-humanPPARgammaLBD wurde in das Plasmid pcDNA3 (Firma Invitrogen) 3'-unterhalb des Cytomegalovirus-Promotors zunächst die für die Aminosäuren 1-147 des Hefetranskriptionsfaktors GAL4 (Genbank-Accession # P04386) kodierende cDNA einkloniert. 3'-unterhalb der GAL4-DNA-Bindungsdomäne wurde anschließend die cDNA der Ligandenbindungsdomäne (LBD) des humanen PPARgamma-Rezeptors kloniert (Aminosäuren 1152-Y475; Accession # g1480099). Klonierung und Sequenzierung des PPARgamma-Expressionsplasmides pcDNA3-GAL4-humanPPARgammaLBD erfolgten wiederum analog wie bei Sambrook J. et. al. beschrieben (Molecular cloning, Cold Spring Harbor Laboratory Press, 1989). Neben dem Luziferase-Reporterplasmid pGL3basic-5xGAL4-TK und dem PPARgamma-Expressionsplasmid pcDNA3-GAL4-humanPPARgammaLBD werden für den zellulären PPARgamma-Test noch das Referenzplasmid pRL-CMV (Firma Promega), sowie das Plasmid pBluescript-SK(+) der Firma Stratagene verwendet. Alle vier Plasmide wurden vor der Transfektion in HEK-Zellen mit einem Plasmidpräparationskit der Firma Qiagen präpariert, der eine möglichst endotoxinfreie Plasmidqualität gewährleistet.

### Durchführung des Tests

Die Aktivität von PPARgamma-Agonisten wird in einem 4-Tagestest bestimmt, der nachfolgend beschrieben ist. Vor der Transfektion werden HEK-Zellen in DMEM-Medium (# 41965-039, Invitrogen) kultiviert, das mit folgenden Zusätzen versetzt ist: 10% FKS (#16000-044, Invitrogen), 1% Penicillin-Streptomycin-Lösung (#15140-122, Invitrogen) und 2 mM L-Glutamin (#25030-024, Invitrogen).

### Tag 1

Zunächst wird Lösung A hergestellt, ein Transfektionsgemisch, das neben DMEM-Medium alle vier bereits beschriebenen Plasmide enthält. Für einen Test werden pro 96-well Mikrotiterplatte folgende Mengen zum Ansetzen von 3 ml Lösung A verwendet: 2622 µl antibiotika- und serumfreies DMEM-Medium (# 41965-039, Invitrogen), 100 µl Referenzplasmid pRL-CMV (1 ng/µl), 100 µl Luziferase-Reporterplasmid pGL3basic-5xGAL4-TK (10 ng/µl), 100 µl PPARgamma-Expressionsplasmid pcDNA3-GAL4-humanPPARgammaLBD (100 ng/µl) und 78 µl Plasmid pBluescript-SK(+) (500 ng/µl). Danach werden pro 96-well Mikrotiterplatte 2 ml Lösung B durch Mischen von 1,9 ml DMEM-Medium (# 41965-039, Invitrogen) mit 100 µl PolyFect-Transfektionsreagens (Firma Qiagen) hergestellt. Anschließend werden 3 ml Lösung A mit 2 ml Lösung B zu 5 ml Lösung C versetzt, durch mehrfaches Pipettieren gründlich gemischt und für 10 min bei Raumtemperatur inkubiert.

80% konfluente HEK-Zellen aus einer 175 cm² großen Zellkulturflasche werden einmal mit 15 ml PBS gewaschen (#14190-094, Invitrogen) und mit 3 ml Trypsinlösung (#25300-054, Invitrogen) für 2 min bei 37°C behandelt. Danach werden die Zellen in 15 ml DMEM-Medium (# 41965-039, Invitrogen) aufgenommen, welches mit 10% FKS (# 16000-044, Invitrogen), 1 % Penicillin-Streptomycin-Lösung (#15140-122, Invitrogen) und 2 mM L-Glutamin (#25030-024, Invitrogen) versetzt ist. Nach dem Zählen der Zellsuspension im Zellzählgerät wird die Suspension auf 250.000 Zellen/ml verdünnt. Für 1 Mikrotiterplatte werden 15 ml dieser Zellsuspension mit 5 ml der Lösung C vermengt. Pro well einer 96 well-Mikrotiterplatte mit klarem Plastikboden (#3610, Coming Costar) werden 200 µl der Suspension ausgesät. Die Platten werden für 24 h in einem Zellkulturbrutschrank bei 37°C und 5 % CO₂ inkubiert.

### Tag 2

Zu testende PPAR-Agonisten werden in einer Konzentration von 10 mM in DMSO gelöst. Diese Stocklösung wird in DMEM-Medium (# 41965-039, Invitrogen) verdünnt, welches mit 2 % Ultroser (#12039-012, Biosepra), 1 % Penicillin-Streptomycin-Lösung (#15140-122, Invitrogen) und 2 mM L-Glutamin (#25030-024, Invitrogen) versetzt ist. Testsubstanzen werden in insgesamt 11 verschiedenen Konzentrationen im Bereich von 10 µM bis 100 pM getestet. Potentere Verbindungen werden in Konzentrationsbereichen von 1 µM bis 10 pM geprüft.

Das Medium der an Tag 1 transfizierten und ausgesäten HEK-Zellen wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Jede Platte wird mit einem Standard PPARgamma-Agonisten belegt, der ebenfalls in 11 verschiedenen Konzentrationen verdünnt wird, um die Funktionsfähigkeit des Tests in jeder Einzelplatte nachzuweisen. Die Testplatten werden für 48 h in einem Brutschrank bei 37 °C und 5 % CO₂ inkubiert.

### Tag 4

Nach dem Absaugen des Mediums werden gemäß den Angaben des Herstellers pro well je 50 µl Dual-Glo™ Reagens (Dual-Glo™ Luciferase Assay System; Firma Promega) zugegeben, um die Zellen zu lysieren und das Substrat für die in den Zellen gebildete Firefly-Luziferase (Photinus pyralis) zur Verfügung zu stellen. Nach einer 10 minütigen Inkubation im Dunkeln, bei Raumtemperatur wird die Firefly-Luziferasevermittelte Chemilumineszenz im Messgerät gemessen (1 sec. Meßzeit/well; Trilux der Firma Wallac). Danach wird pro well je 50 µl des Dual-Glo™ Stop & Glo Reagens (Dual-Glo™ Luciferase Assay System; Firma Promega) zugegeben, um die Aktivität der Firefly-Luziferase abzustoppen und das Substrat für die vom Referenzplasmid pRL-CMV aus exprimierten Renilla-Luciferase zur Verfügung zu stellen. Nach einer weiteren 10 minütigen Inkubation im Dunkeln bei Raumtemperatur wird erneut für 1 sec/well die durch die Renilla-Luziferase vermittelte Chemilumineszenz im Messgerät gemessen.

### Auswertung

Die Rohdaten des Lumineszenzmeßgerätes werden in ein Microsoft Excel-File transferiert. Für jeden Meßpunkt, der sich von einem well der Mikrotiterplatte ableitet, wird der Quotient Firefly/Renilla-Luciferaseaktivität bestimmt. Aus den Quotienten werden die Dosis-Wirkungskurven und EC50-Werte von PPAR-Agonisten mit dem Program XL.Fit nach Vorgabe des Herstellers (Firma IDBS) berechnet.
Mit den in dieser Anmeldung beschriebenen PPAR-Agonisten wurden PPARgamma-EC50-Werte im Bereich von 6nM bis >10 µM gemessen.

Die nachstehend aufgeführten Beispiele dienen zur Erläuterung der Erfindung, ohne diese jedoch einzuschränken.

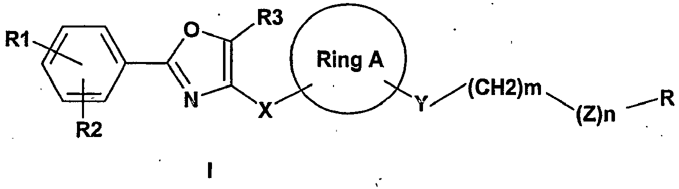

| | R1 | R2 | R3 | X | Y | Ring A | m | n | Z | R |
|---|---|---|---|---|---|---|---|---|---|---|
| I | 3-Me | H | Me | CH2O | O | 1,3-Cy | 3 | 0 | - | 5-Tetrazol |
| II | 3-Me | H | Me | CH2O | O | 1,3-Cy | 3 | 0 | - | NH-SO2CF3 |
| III | 3-Me | H | Me | CH2O | O | 1,3-Cy | 2 | 0 | - | 5-Tetrazol |
| IV | 3-Me | H | Me | CH2O | O | 1,3-Cy | 2 | 0 | - | NH-SO2CF3 |
| V | 3-Me | H | Me | CH2O | O | 1,3-Cy | 2 | 0 | - | 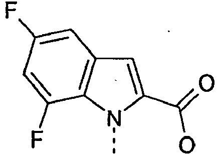 |
| VI | 3-Me | H | Me | CH2O | O | 1,3-Cy | 2 | 0 | - | 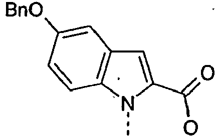 |
| VII | 3-Me | H | Me | CH2O | O | 1,3-Cy | 2 | 0 | - | 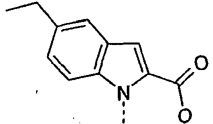 |
| VIII | 3-Me | H | Me | CH2O | O | 1,3-Cy | 2 | 0 | - | 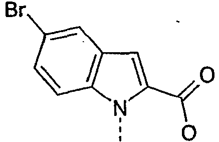 |
| IX | 3-Me | H | Me | CH2O | O | 1,3-Cy | 2 | 0 | - | 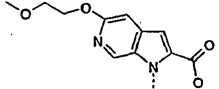 |
| X | 3-Me | H | Me | CH2O | O | 1,3-Cy | 2 | 0 | - | 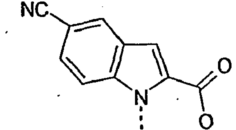 |
| XI | 3-Me | H | Me | CH2O | O | 1,3-Cy | 2 | 0 | - | 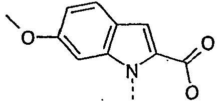 |
| XII | 3-Me | H | Me | CH2O | O | 1,3-Cy | 2 | 0 | - | 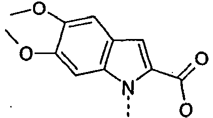 |
| XIII | 3-Me | H | Me | CH2O | O | 1,3-Cy | 2 | 0 | - | 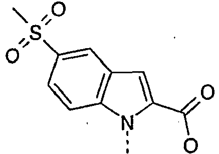 |
| XIV | 3-Me | H | Me | CH2O | O | 1,3-Cy | 2 | 1 | O | 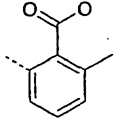 |
| XV | 3-Me | H | Me | CH2O | O | 1,3-Cy | 2 | 1 | O | 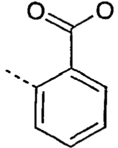 |
| XVI | 3-Me | H | Me | CH2O | O | 1,3-Cy | 2 | 1 | O | 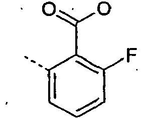 |
| XVI | 3-Me | H | Me | CH2O | O | 1,3-Cy | 2 | 1 | O | 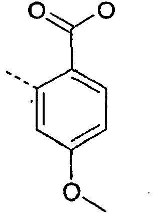 |
| XVIII | 3-Me | H | Me | CH2O | O | 1,3-Cy | 2 | 1 | O | 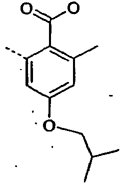 |
| XIX | 3-Me | H | Me | CH2O | O | 1,3-Cy | 2 | 1 | O | 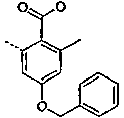 |
| XX | 3-Me | H | Me | CH2O | O | 1,3-Cy | 2 | 1 | O | 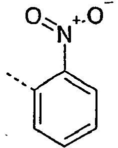 |
| XXI | 3-Me | H | Me | CH2O | O | 1,3-Cy | 2 | 1 | O | 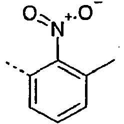 |
| XXII | 3-Me | H | Me | CH2O | O | 1,3-Cy | 2 | 1 | O | 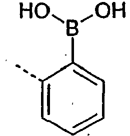 |
| XXIII | 3-Me | H | Me | CH2O | O | 1,3-Cy | 2 | 1 | O | 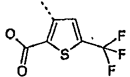 |
| XXIV | 3-Me | H | Me | CH2O | O | 1,3-Cy | 1 | 0 | - | 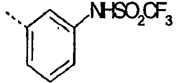 |
| XXV | 3-Me | H | Me | CH2O | O | 1,3-Cy | 1 | 0 | - | 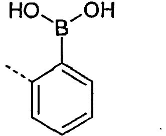 |
| XXVI | 4-Me | H | Me | CH2O | - | 1,3-Cy | 1 | 0 | - | 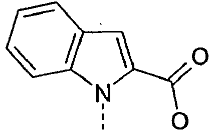 |
| XXVII | 4-Me | H | Me | CH2O | - | 1,3-Cy | 1 | 0 | - | 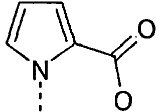 |
| XXVIII | 3-Ome | H | Me | CH2O | - | 1,3-Cy | 1 | 0 | - | 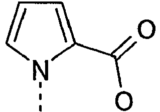 |
| XXIX | 4-Me | H | Me | CH2O | - | 1,3-Cy | 1 | 1 | O | 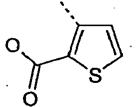 |
| XXX | 4-Me | H | Me | CH2O | - | 1,3-Cy | 1 | 1 | O | 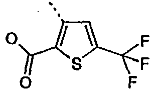 |
| XXXI | 4-Me | H | Me | CH2O | - | 1,3-Cy | 1 | 1 | O | 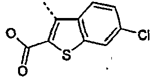 |

| | | | | | | | | | | |
|---|---|---|---|---|---|---|---|---|---|---|
| Ring A: 1,3 Cy = cis Cyclohexan-1,3-diyl mit der Stereochemie nach Cahn-Ingold-Prelog, wie sie in den Beispielen angegeben ist. R: ----- gibt die Verknüpfung an. | | | | | | | | | | |

Die Verbindungen der allgemeinen Formel I können entsprechend den folgenden Reaktionsschemata erhalten werden:

### Syntheseschema I: Darstellung der zentralen Intermediate 6 und 7

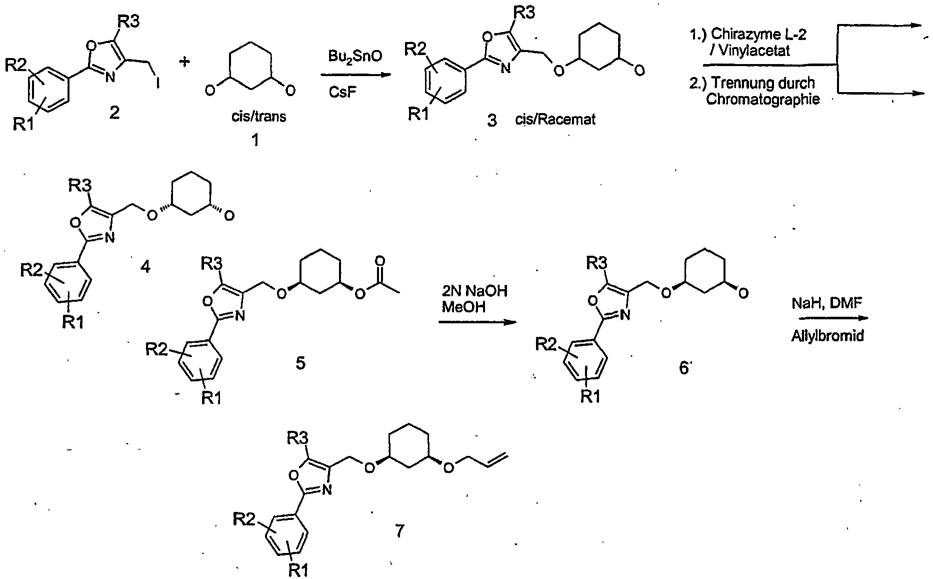

### Syntheseschema II: Funktionalisierung der Allylgruppe in 7

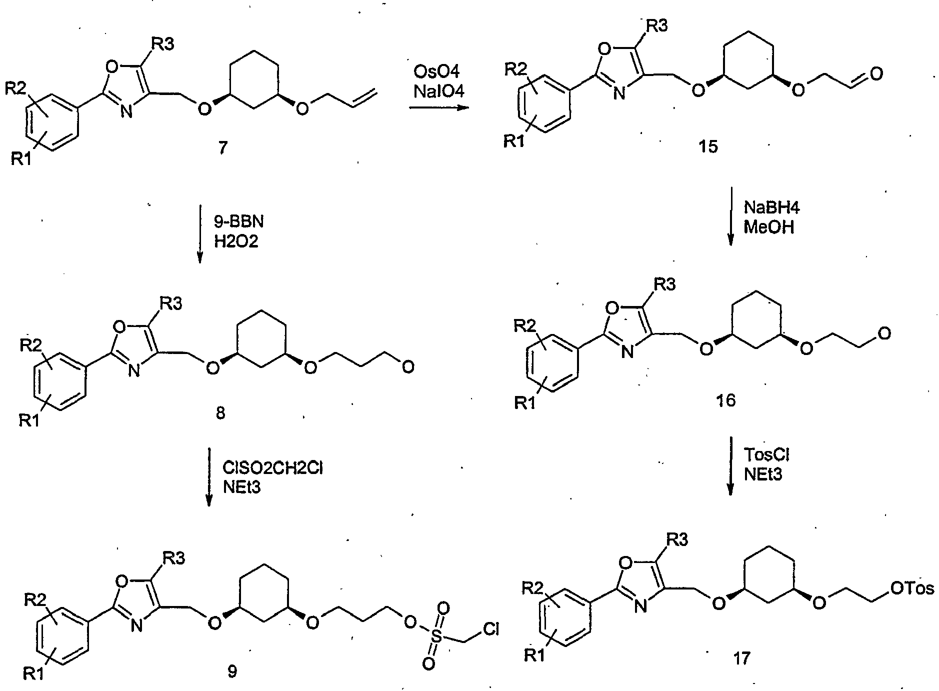

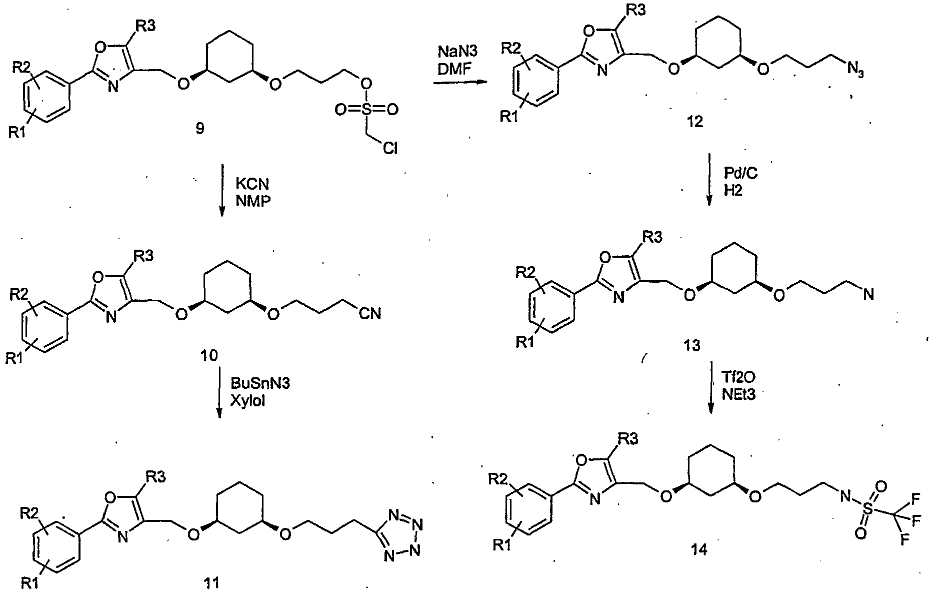

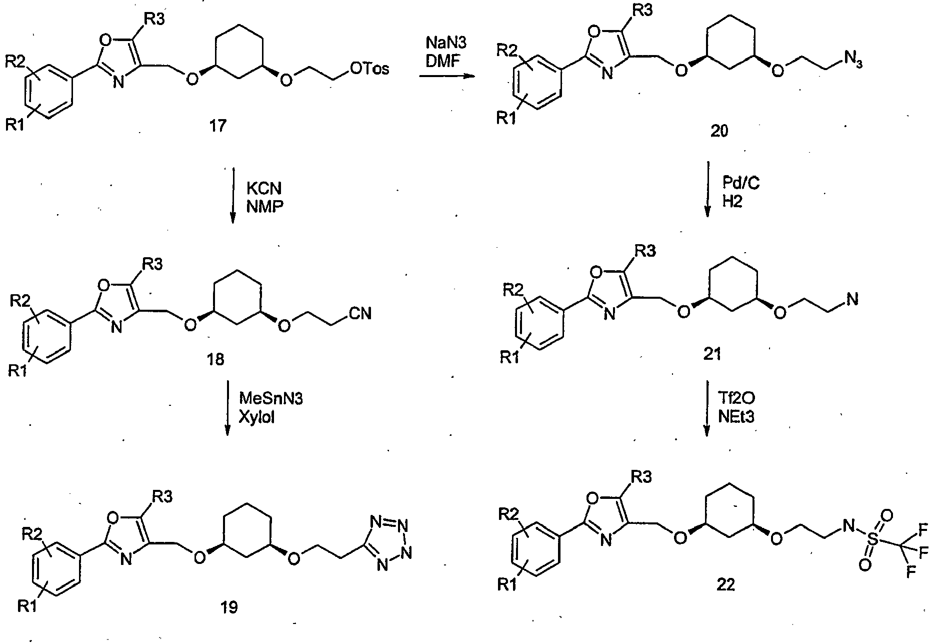

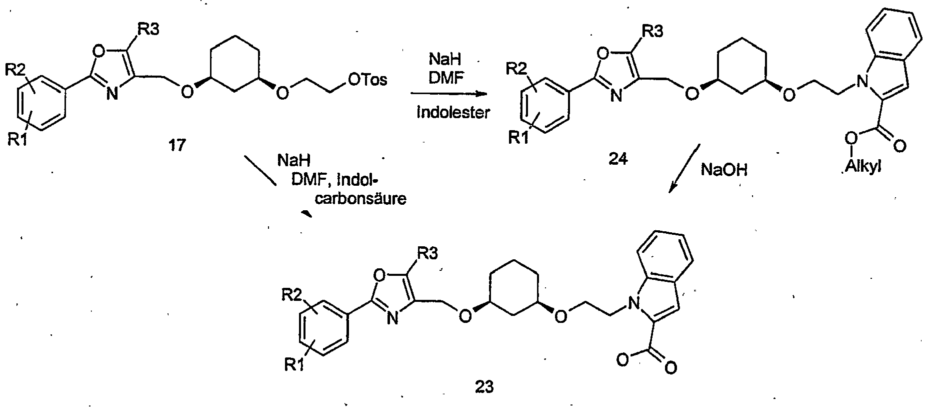

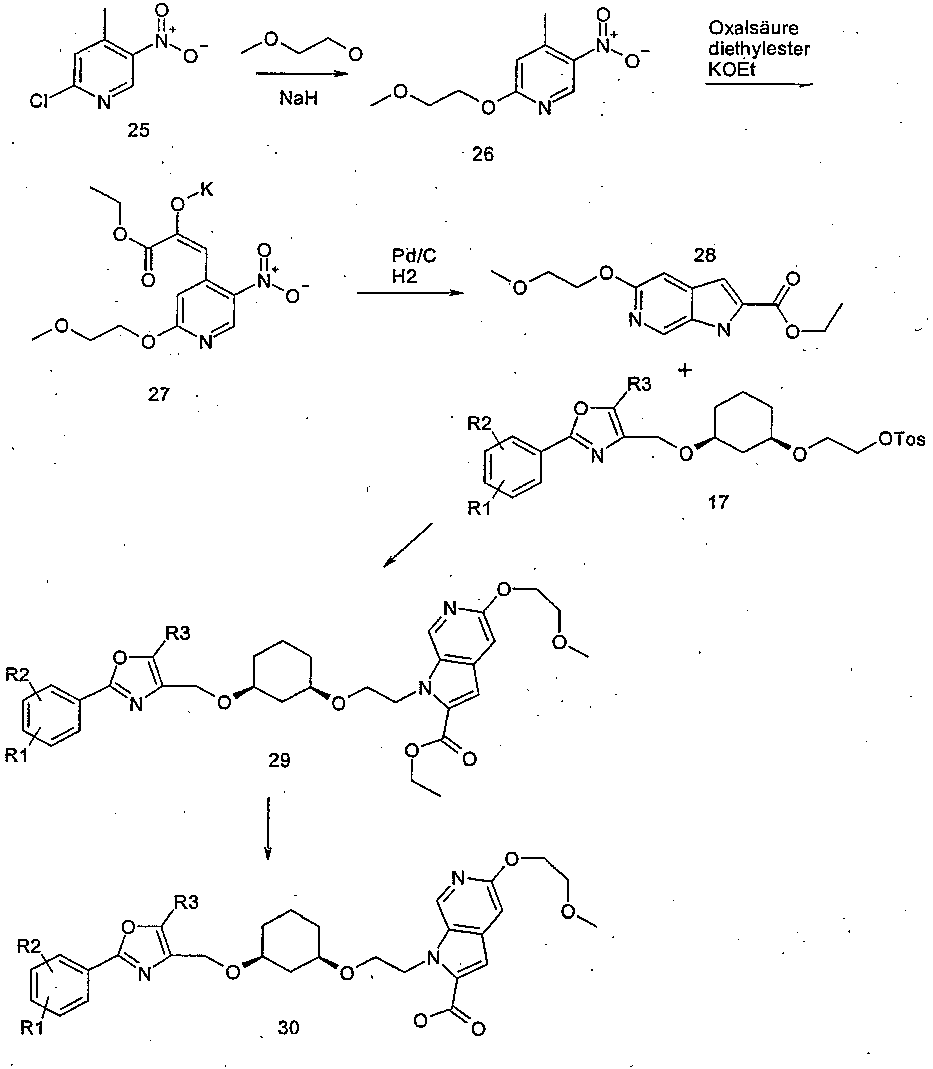

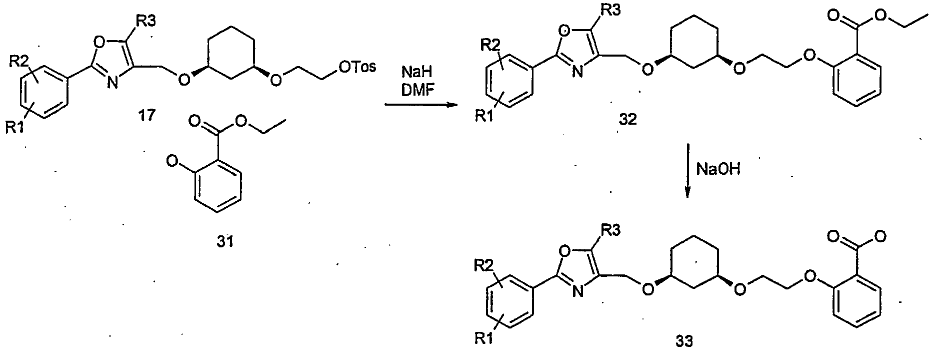

### Syntheseschema III: Umsetzung von 6 mit funktionalisierten Benzylbromiden

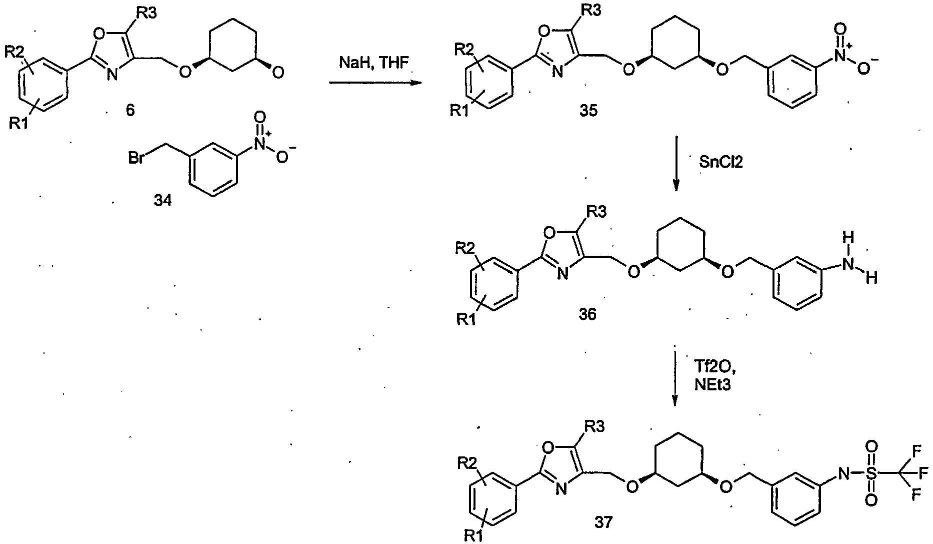

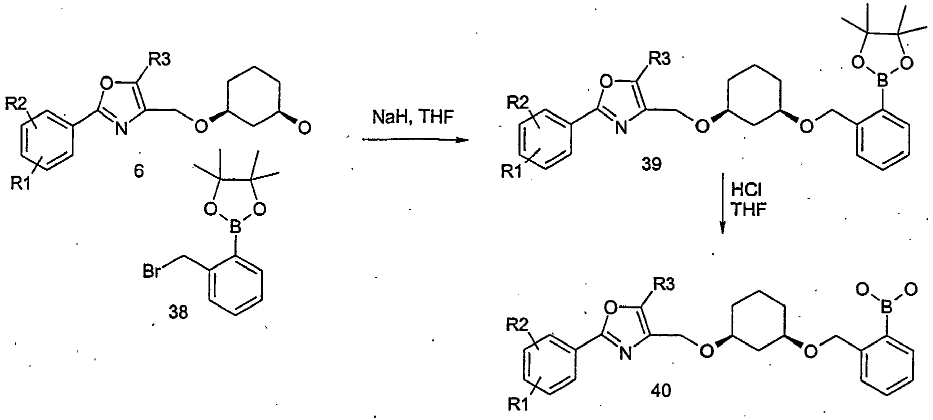

### Syntheseschema IV:

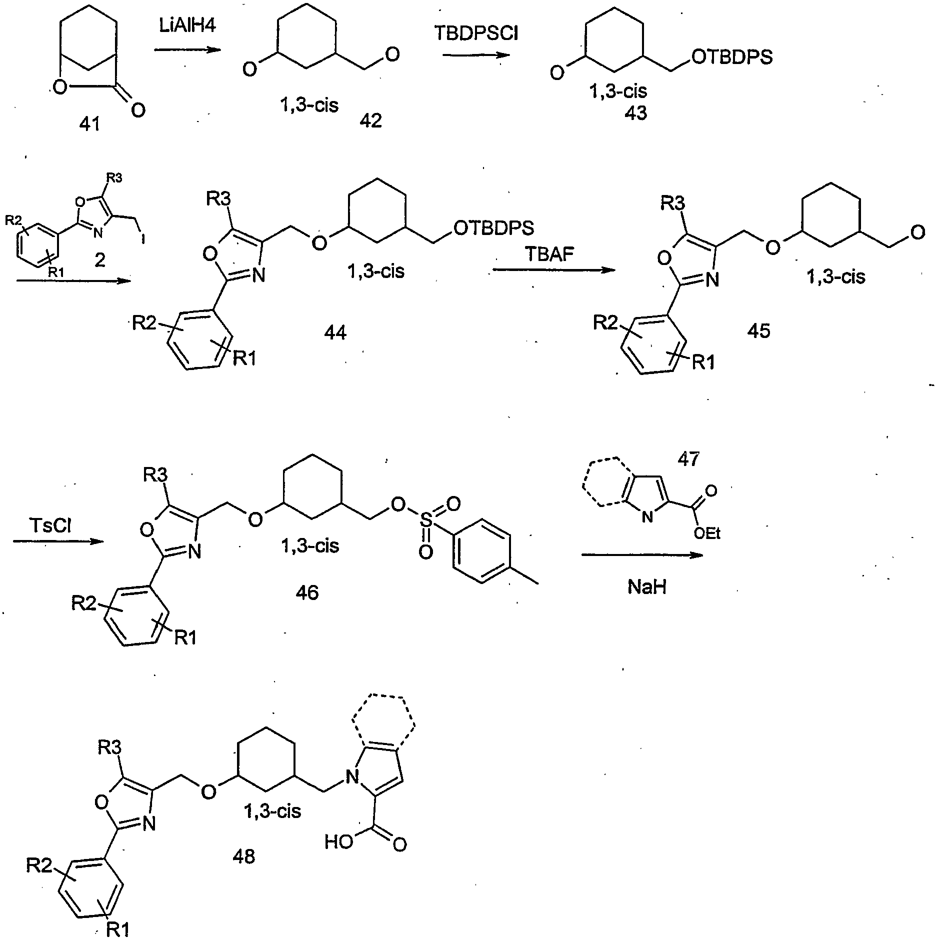

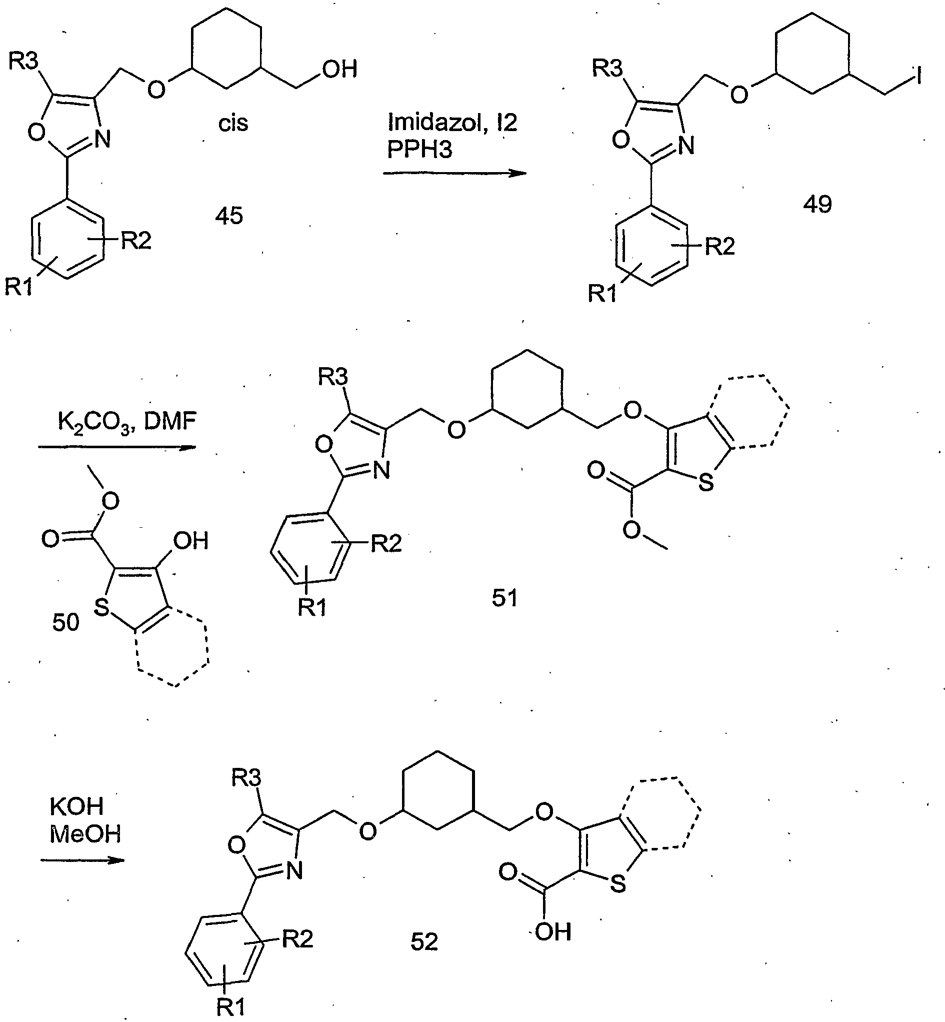

Dabei wird die Komponente 1 zunächst mit Dibutylzinnoxid in Toluol mehrere Stunden am Wasserabscheider erhitzt und dann unter Zusatz von Dimethylformamid, Cäsiumfluorid und Iodid 2 durch mehrstündiges Rühren bei Raumtemp. zu Verbindungen der allg. Struktur 3 umgesetzt, worin R1, R2, R3, die oben beschriebenen Bedeutungen haben.

Die Verbindung der allgemeinen Formel 3 unter Verwendung von Chirazym L2 und Vinylacetat umgesetzt, dabei entstehen die Verbindungen 4 und 5, von denen 5, nach Trennung, mit Alkalihydroxyden zu Verbindungen der allg. Struktur 6 umgesetzt wird, worin R1, R2, R3, die oben beschriebenen Bedeutungen haben.

Die Verbindung der allgemeinen Formel 6 wird unter Verwendung von starken Basen; z. B. Natriumhydrid in einem aprotischen Lösungsmittel deprotoniert, und mit ungesättigten Bromiden, z. B. Allylbromid, zu Verbindungen der allg. Struktur 7 umgesetzt, worin R1, R2, R3, die oben beschriebenen Bedeutungen haben.

Die Verbindung der allgemeinen Formel 7 wird unter Verwendung von Hydroborierungsreagenzien, wie z. B. 9-BBN, und nachfolgender Behandlung mit alkalischem Wasserstoffperoxid zu Verbindungen der allg. Struktur 8 umgesetzt, die mit Sulfonsäurechloriden, z. B. Chlormethylsulfonylchlorid, zu Verbindungen der allg. Struktur 9 umgesetzt wird, worin R1, R2, R3, die oben beschriebenen Bedeutungen haben.

Alternativ kann die Verbindung der allgemeinen Formel 7 unter Verwendung von Osmiumtetroxid und Natriumperiodat zu dem Aldehyd der allg. Struktur 15 umgesetzt werden, der mit komplexen Hydriden, z. B. Natriumborhydrid, zu Verbindungen der allg. Struktur 16 umgesetzt wird. Die weitere Umsetzung von Verbindung der allgemeinen Formel 16 mit Sulfonsäurechloriden, z. B. Toluolsulfonsäurechlorid, ergibt Verbindungen der allg. Struktur 17, worin R1, R2, R3, die oben beschriebenen Bedeutungen haben.

Die Verbindung der allgemeinen Formel 9 wird unter Verwendung von Natriumcyanid in aprotischen Lösungsmitteln, wie z. B. NMP, zu Verbindungen der allg. Struktur 10 umgesetzt, die mit einem Metallazid, z. B. Tributylzinnazid, zu Verbindungen der allg. Struktur 11 umgesetzt wird, worin R1, R2, R3, die oben beschriebenen Bedeutungen haben.

Alternativ kann die Verbindung der allgemeinen Formel 9 unter Verwendung von Natriumazid in aprotischen Lösungsmitteln, wie z. B. NMP, zu Verbindungen der allg. Struktur 12 umgesetzt werden, die durch katalytische Hydrierung, z. B. mit Palladium auf Kohle, zu den Verbindungen der allg. Struktur 13 umgesetzt werden. Die weitere Umsetzung von Verbindung der allgemeinen Formel 13 mit Sulfonsäurechloriden, z. B. Trifluormethansulfonsäureanhyrid, ergibt Verbindungen der allg. Struktur 14, worin R1, R2, R3, die oben beschriebenen Bedeutungen haben.

Die Verbindung der allgemeinen Formel 17 wird unter Verwendung von Natriumcyanid in aprotischen Lösungsmitteln, wie z. B. NMP, zu Verbindungen der allg. Struktur 18 umgesetzt, die mit einem Metallazid, z. B. Tributylzinnazid, zu Verbindungen der allg. Struktur 19 umgesetzt wird, worin R1, R2, R3, die oben beschriebenen Bedeutungen haben.

Alternativ kann die Verbindung der allgemeinen Formel 17 unter Verwendung von Natriumazid in aprotischen Lösungsmitteln, wie z. B. NMP, zu Verbindungen der allg. Struktur 20 umgesetzt werden, die durch katalytische Hydrierung, z. B. mit Palladium auf Kohle, zu den Verbindungen der allg. Struktur 21 umgesetzt werden. Die weitere Umsetzung von Verbindung der allgemeinen Formel 21 mit Sulfonsäurechloriden, z. B. Trifluormethansulfonsäureanhyrid, ergibt Verbindungen der allg. Struktur 22, worin R1, R2, R3, die oben beschriebenen Bedeutungen haben.

Stickstoffhaltige aromatische Heterocyclen z. B. Pyrrol, Indol, Azaindol, werden durch Behandlung mit starken Basen, z. B. Natriumhydrid, in polar aprotischen Lösungsmitteln, wie z. B. DMF, in die entsprechenden Natriumsalze überführt und mit einer Verbindung der allgemeinen Formel 17 zu Verbindungen der allg. Struktur 23 und 24 umgesetzt. Die Verbindung der allgemeinen Formel 24 wird unter Verwendung von Alkalihydroxiden zu Verbindungen der allg. Struktur 23 umgesetzt, worin R1, R2, R3, die oben beschriebenen Bedeutungen haben.

Azaindol-2-carbonsäurester des Typs 28 werden hergestellt durch nukloephile aromatische Substitution an 2-Chlor-Nitropyridinen (25) mit Alkoholaten und nachfolgender Kondensation mit Oxalsäureestern. Durch reduktive Zyklisierung unter Wasserstoff-Atmosphäre erhält man Azaindol-2-carbonsäurester des Typs 28, die wie oben beschrieben mit einer Verbindung der allgemeinen Formel 17 zu Verbindungen der allg. Struktur 29 umgesetzt werden können. Nach Verseifung des Esters, die zu Verbindungen der allg. Struktur 22 ähnlichen Verbindungen der allg. Struktur 30.

Mit einer Hydroxygruppe substituierte Benzoesäureester der allgemeinen Formel 31 und heterocyclische Carbonsäureester, z. B. Thiophen und Furan, werden durch Behandlung mit starken Basen, z. B. Natriumhydrid, in polar aprotischen Lösungsmitteln, wie z. B. DMF, in die entsprechenden Natriumsalze überführt und mit einer Verbindung der allgemeinen Formel 17 zu Verbindungen der allg. Struktur 32 umgesetzt. Die Verbindung der allgemeinen Formel 32 wird unter Verwendung von Alkalihydroxiden zu Verbindungen der allg. Struktur 33 umgesetzt, worin R1, R2, R3, die oben beschriebenen Bedeutungen haben.

Die Verbindung der allgemeinen Formel 6 wird unter Verwendung von starken Basen, z. B. Natriumhydrid in einem aprotischen Lösungsmittel deprotoniert, und mit Benzylbromiden der allgemeinen Formel 34 zu Verbindungen der allg. Struktur 35 umgesetzt. Die Verbindung der allgemeinen Formel 35 wird unter Verwendung eines Reduktionsmittels, z. B. Zinn(II)chlorid, in einem aprotischen Lösungsmittel zu Verbindungen der allgemeinen Formel 36 umgesetzt, die mit Trifluormethan-sulfonsäureanhydrid in die Sulfonamide der allgemeinen Formel 37, worin R1, R2, R3, die oben beschriebenen Bedeutungen haben.

Die Verbindung der allgemeinen Formel 6 wird unter Verwendung von starken Basen, z. B. Natriumhydrid in einem aprotischen Lösungsmittel deprotoniert, und mit Benzylbromiden der allgemeinen Formel 38 zu Verbindungen der allg. Struktur 39 umgesetzt. Die Verbindung der allgemeinen Formel 39 wird mit wässriger Säure zu Verbindungen der allgemeinen Formel 40 umgesetzt, worin R1, R2, R3, die oben beschriebenen Bedeutungen haben.

Das Lacton 41 wird mit Lithiumalanat zum Diol 42 reduziert, das selektiv mono-Silyl geschützt werden kann (43). Die Verbindung der allgemeinen Formel 43 wird unter Verwendung von starken Basen, z. B. Natriumhydrid in einem aprotischen Lösungsmittel deprotoniert, und mit Phenyloxazoyliodiden der allgemeinen Formel 2 zu Verbindungen der allg. Struktur 44 umgesetzt. Die Verbindung der allgemeinen Formel 45 wird mit Fluorid, z. B. TBAF, desilyliert , wobei man Verbindungen der allgemeinen Formel 45 erhält, die man mit Sulfonsäurechloriden, z. B. Toluolsulfonsäurechlorid, zu Verbindungen der allgemeinen Formel 46 umsetzt, worin R1, R2, R3, die oben beschriebenen Bedeutungen haben.

Stickstoffhaltige aromatische Heterocyclen der allgemeinen Formel 47, z. B. Pyrrol, Indol, Azaindol, werden durch Behandlung mit starken Basen, z. B. Natriumhydrid, in polar aprotischen Lösungsmitteln, wie z. B. DMF, in die entsprechenden Natriumsalze überführt und mit einer Verbindung der allgemeinen Formel 48 umgesetzt, worin R1, R2, R3, die oben beschriebenen Bedeutungen haben.

Mit einer Hydroxygruppe substituierte heterocyclische Carbonsäureester, z. B: Thiophen und Benzothiophen, werden in Gegenwart von schwachen Basen, z. B. Kaliumcarbonat, in polar aprotischen Lösungsmitteln, wie z. B. DMF, in die deprotoniert und mit einer Verbindung der allgemeinen Formel 49 zu Verbindungen der allg. Struktur 51 umgesetzt. Die Verbindung der allgemeinen Formel 51 wird unter Verwendung von Alkalihydroxiden zu Verbindungen der allg. Struktur 52 umgesetzt, worin R1, R2, R3, die oben beschriebenen Bedeutungen haben.

Andere Verbindungen der Formel I können entsprechend oder nach bekannten Verfahren hergestellt werden.

Die nachstehend aufgeführten Beispiele und Herstellungsmethoden dienen zur Erläuterung der Erfindung, ohne diese jedoch einzuschränken.

### rac-3-(cis-5-Methyl-2-m-tolyl-oxazol-4-ylmethoxy)-cyclohexanol

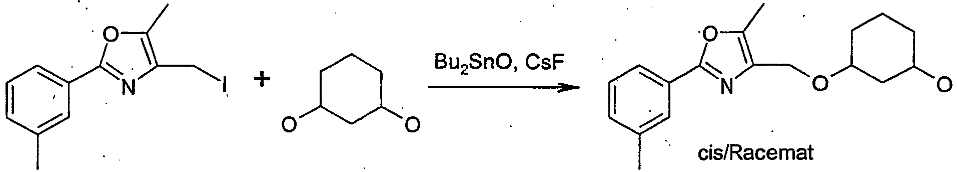

21.7 g 1,3-Cyclohexandiol werden mit 30.3 g Dibutylzinnoxid in 450 ml Toluol gelöst und unter Rückfluss am Wasserabscheider zum Sieden erhitzt. Das Reaktionsvolumen wird während der Reaktionsdauer auf die Hälfte reduziert. Nach 3 Std. wird das Reaktionsgemisch auf Raumtemp. gekühlt und mit 300 ml DMF, 29 g 4-iodomethyl-5-methyl-2-m-tolyl-oxazol und 23.5 g Cäsiumfluorid versetzt. Man rührt 18 Std. bei Raumtemp. nach. Das Reaktionsgemisch wird durch Zugabe von Ethylacetat verdünnt und mit gesättigter NaCl-Lösung gewaschen. Die organische Phase wird über Magnesiumsulfat getrocknet, das Lösungsmittel im Vakuum entfernt und der Rückstand durch Flash-Chromatographie an Kieselgel (n-Heptan/Ethylacetat = 10:1 -> 1:4) gereinigt. Man erhält 58 g des Racemats cis-3-(5-Methyl-2-m-tolyl-oxazol-4-ylmethoxy)-cyclohexanol als gelblichen Feststoff, der aus n-Heptan/Ethylacetat umkristallisiert wird. C₁₈H₂₃NO₃ (301.39), MS (ESI): 302 (M + H⁺).

### 3-((1R,3S)-cis-5-Methyl-2-m-tolyl-oxazol-4-ylmethoxy)-cyclohexanol

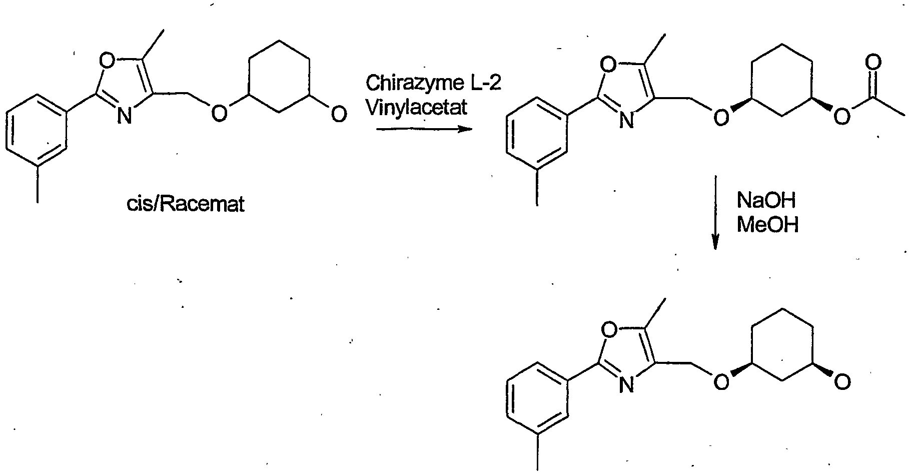

25 g racemisches cis-3-(5-Methyl-2-m-tolyl-oxazol-4-ylmethoxy)-cyclohexanol werden in 320 ml Vinylacetat gelöst und mit 1,3 g Chirazyme L-2 Lyo (Boehringer Mannheim). versetzt. Nach ca dreistündigem Rühren bei Raumtemp. (LC-MS Kontrolle auf 40-45% Umsatz) wird das Enzym abfiltriert, mit Ethylacetat nachgewaschen und das Lösungsmittel im Vakuum entfernt. Der Rückstand wird durch Flash-Chromatographie an Kieselgel (n-Heptan/Ethylacetat = 3:1) gereinigt. Man erhält 8 g des Acetats Essigsäure-(1R,3S)-3-(5-methyl-2-m-tolyl-oxazol-4-ylmethoxy)-cyclohexyl ester als farbloses Öl. Q₂₀H₂₅NO₄ (343.43), MS (ESI): 344 (M + H⁺), Man nimmt das Acetat Essigsäure-(1 R,3S)-3-(5-methyl-2-m-tolyl-oxazol-4-ylmethoxy)-cyclohexyl ester in 170 ml Methanol auf und rührt nach Zugabe von 27 ml 2N NaOH für eine Stunde bei Raumtemp.. Der größte Teil des Lösungsmittels wird im Vakuum entfernt. Nach Zugabe von je 150 ml Wasser und Ethylacetat, wird die org. Phase mit NaCl-Lösung gewaschen. Die organische Phase wird über Magnesiumsulfat getrocknet, das Lösungsmittel im Vakuum entfernt. Man erhält 6,7 g 3-((1R,3S)-5-Methyl-2-m-tolyl-oxazol-4-ylmethoxy)-cyclohexanol als gelblichen Feststoff. C₁₈H₂₃NO₃ (301.39), MS (ESI): 302 (M + H⁺):

### 4-((1R,3S)-3-Allyloxy-cyclohexyloxymethyl)-5-methyl-2-m-tolyl-oxazol

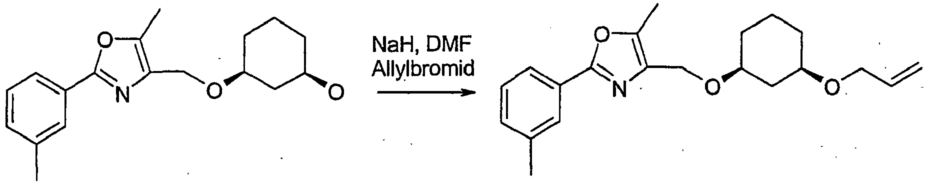

Zu einer Lösung von 2.2 g 3-((1R,3S)-5-Methyl-2-m-tolyl-oxazol-4-ylmethoxy)-cyclohexanol in 30 ml Dimethylformamid werden bei Raumtemp. 470 mg 60-proz. Natriumhydrid-Suspension gegeben und 20 min bei Raumtemperatur gerührt. Anschließend werden 1,36 ml Allylbromid addiert man rührt bei 40 °C bis zum vollständigen Umsatz, eventuell werden weiteres Natriumhydrid und Allybromid zugegeben. Bei vollständigen Umsatz (LC-MS Kontrolle) werden 100 ml Ethylacetat und 150 ml ges NaCl-Lösung zugegeben. Die organische Phase wird über Magnesiumsulfat getrocknet, die Lösungsmittel im Vakuum entfernt und der Rückstand durch Flash-Chromatographie an Kieselgel (n-Heptan/Ethylacetat = 3:1) gereinigt. Man erhält 2.3 g 4-((1R,3S)-3-Allyloxy-cyclohexyloxymethyl)-5-methyl-2-m-tolyl-oxazol5 als farbloses Öl. C₂₁H₂₇NO₃ (341.45), MS (ESI): 342 (M + H⁺).

### 3-(1R,3S)-[3-(5-Methyl-2-m-tolyl-oxazol-4-ylmethoxy)-cyclohexyloxy]-propan-1-ol

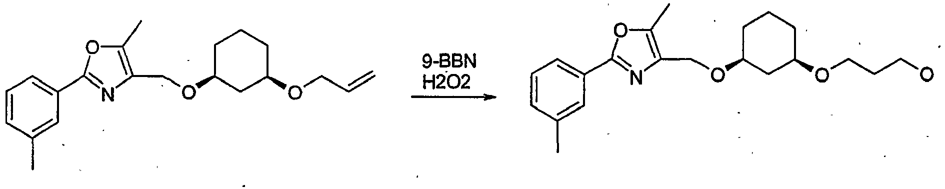

3.3 g 4-((1 R,3S)-3-(Allyloxy-cyclohexyloxymethyl)-5-methyl-2-m-tolyl-oxazol werden in 70 ml trockenem THF gelöst und bei Raumtemp. mit 20 ml 9-BBN 0,5 M in THF versetzt. Man rührt 4 h bei dieser Temperatur und gibt nacheinander 10 ml Wasser, 10 ml 2 N NaOH und 5 ml H₂O₂:30%ig zu. Man rührt 18 h bei Raumtemp. und erhitzt nachfolgend für 1 h auf 40°C. Nach dem Erkalten gibt man 50 ml Wasser zu, extrahiert zweimal mit 100 ml Methyl-tert.-butylether und wäscht die vereinigten org. Phasen mit ges. NaCl-Lösung. Die organischen Phasen werden über Natriumsulfat getrocknet und das Lösungsmittel im Vakuum entfernt. Der Rückstand wird durch Flash-Chromatographie an Kieselgel (n-Heptan/Ethylacetat = 1:2) gereinigt. Man erhält 1,56 g 3-(1R,3S)-[3-(5-Methyl-2-m-tolyl-oxazol-4-ylmethoxy)-cyclohexyloxy]-propan-1-ol als farbloses Öl. C₂₁H₂₉NO₄ (359.47), MS (ESI): 360 (M + H⁺).

### 4-((1R,3S)-Chloro-methanesulfonsäure-3-[3-(5-methyl-2-m-tolyl-oxazol-4-ylmethoxy)-cyclohexyloxy]- propyl ester

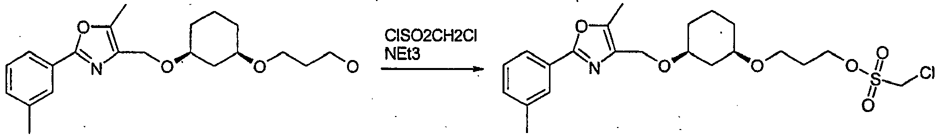

1.56 g 3-(1 R,3S)-[3-(5-Methyl-2-m-tolyl-oxazol-4-ylmethoxy)-cyclohexyloxy]-propan-1-ol werden in 15 ml Dichlormethan gelöst und bei 0°C mit 0,7 ml Pyridin und 0,45 ml Chlormethansulfonsäürechlorid versetzt. Nach fünfstündigem Rühren bei dieser Temperatur wird das Gemisch auf Eis/1 N HCl gegossen und mit Dichlormetan extrahiert. Man trocknet über Natriumsulfat und entfernt das Lösungsmittel im Vakuum. Der Rückstand wird durch Flash-Chromatographie an Kieselgel (n-Heptan/Ethylacetat = 1:1) gereinigt. Man erhält 1,8 g des 4-((1R,3S)-Chloro-methanesulfonsäure-3-[3-(5-methyl-2-m-tolyl-oxazol-4-ylmethoxy)-cyclohexyloxy]-propyl ester als farbloses Öl. C₂₂H₃₀CINO₆S (472.00), MS (ESI): 473 (M + H⁺),

### 2-((1R,3S)-[3-(5-Methyl-2-m-tolyl-oxazol-4-ylmethoxy)-cyclohexyloxy]-ethanol

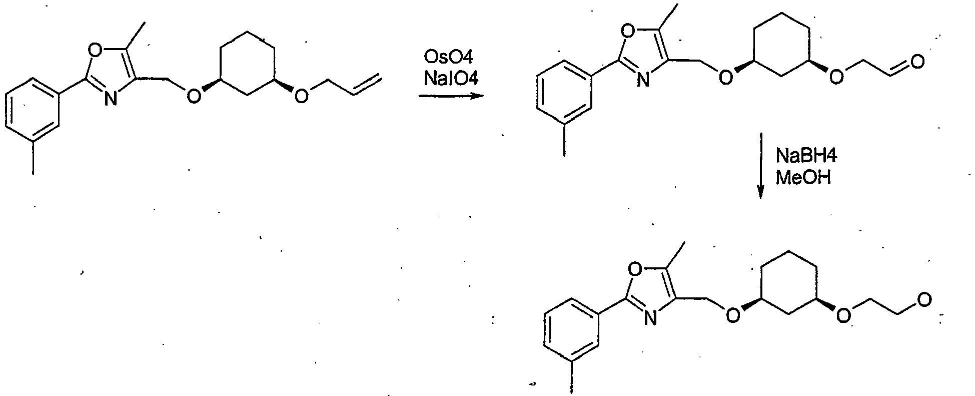

2.3 g 4-((1R,3S)-3-(Allyloxy-cyclohexyloxymethyl)-5-methyl-2-m-tolyl-oxazol werden in 65 ml Methyl-tert.-butylether gelöst und bei 0°C mit 65 ml Wasser, 4.4 g Natriummetaperiodat und 3,3 ml einer 2,5%igen Lösung von Osmiumtetroxid in tert.-Butanol versetzt. Nach 20 min wird langsam auf 40°C erwärmt. Nach 2 h wurden weitere 700 mg Natriummetaperiodat zugegeben und 2 h bei 45°C gerührt. Man addierte 140 ml ges Natriumthiosulfatlösung und extrahierte mit Methyl-tert.-butylether. Die vereinigten organischen Phasen werden über Natriumsulfat getrocknet und das Lösungsmittel im Vakuum entfernt. Man erhält den 2-((1R,3S)-[3-(5-Methyl-2-m-tolyl-oxazol-4-ylmethoxy)-cyclohexyloxy]-acetaldehyd als farbloses Öl. C₂₀H₂₅NO₄ (343.43), MS (ESI): 344 (M + H⁺). Der 2-((1R,3S)-[3-(5-Methyl-2-m-tolyl-oxazol-4-ylmethoxy)-cyclohexyloxy]-acetaldehyd wird als Rohprodukt in 60 ml trockenem Methanol aufgenommen und mit 300 mg Natriumborhydrid versetzt. Nach 1,5 h wird mit 3 ml Aceton gequencht und aufkonzentriert. Man nimmt den Rückstand auf in 50 ml EE /50 ml ges Natriumhydrogencarbonat-Lösung und trocknet die org. Phase über Natriumsulfat. Nach Entfernen des Lösungsmittels im Vakuum wird der Rückstand durch Flash-Chromatographie an Kieselgel (n-Heptan/Ethylacetat = 1:3) gereinigt. Man erhält 1.6 g 2-((1R,3S)-[3-(5-Methyl-2-m-tolyl-oxazol-4-ylmethoxy)-cyclohexyloxy]-ethanol als farbloses Öl. C₂₀H₂₇NO₄ (345.44), MS (ESI): 346 (M + H⁺).

### Toluol-4-sulfonsäure((1R,3S)-[3-(5-methyl-2-m-tolyl-oxazol-4-ylmethoxy)-cyclohexyloxy]-ethyl ester

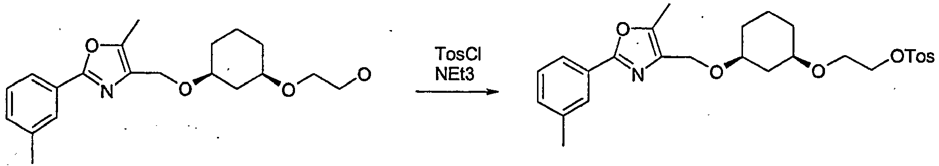

1.6 g 2-((1R,3S)-[3-(5-Methyl-2-m-tolyl-oxazol-4-ylmethoxy)-cyclohexyloxy]-ethanol werden in einer Mischung aus 30 ml Dichlormethan und 3.6 ml Pyridin gelöst und 1,2 g 4-Tosylchlorid sowie 50 mg Dimethylaminopyridin zugegeben. Man rührt 18 h bei Raumtemp., gießt anschließend auf 2N HCl/Eis und extrahiert die wässrige Phase mit 50 ml Dichlormethan. Die vereinigten organischen Phasen werden über Natriumsulfat getrocknet und das Lösungsmittel im Vakuum entfernt. Der Rückstand wird durch Flash-Chromatographie an Kieselgel (n-Heptan/Ethylacetat = 2:1) gereinigt. Man erhält Toluol-4-sulfonsäure((1R,3S)-[3-(5-methyl-2-m-tolyl-oxazol-4-ylmethoxy)-cyclohexyloxy]-ethyl ester als farbloses Öl. C₂₇H₃₃NO₆S (513.66), MS (ESI): 514 (M + H⁺).

### Beispiel I

### (1 R,3S)-5-{3-[3-(5-Methyl-2-m-tolyl-oxazol-4-ylmethoxy)-cyclohexyloxy]-propyl}-2H-tetrazol

### 1R,3S)-4-[3-(5-Methyl-2-m-tolyl-oxazol-4-ylmethoxy)-cyclohexyloxy]-butyronitril

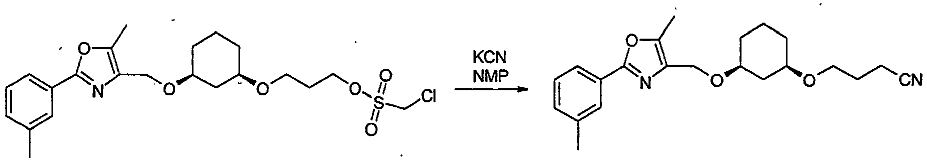

472 mg des 4-((1 R,3S)-Chloro-methanesulfonsäure 3-[3-(5-methyl-2-m-tolyl-oxazol-4-ylmethoxy)-cyclohexyloxy]-propyl ester werden in 3 ml N-Methylpyrrolidon gelöst und 200 mg Kaliumcyanid sowie 30 mg Tetrabutylammoniumiodid zugegeben. Man rührt 3 h bei 60°C und nimmt nach dem Erkalten in 20 ml Ethylacetat/20 ml mit ges. NaCl-Lösung auf. Die organische Phase wird über Natriumsulfat getrocknet und das Lösungsmittel im Vakuum entfernt. Der Rückstand wird durch Flash-Chromatographie an Kieselgel (n-Heptan/Ethylacetat = 1:1) gereinigt. Man erhält das (1R,3S)-4-[3-(5-Methyl-2-m-tolyl-oxazol-4-ylmethoxy)-cyclohexyloxy]-butyronitril als farbloses Öl. C₂₂H₂₈N₂O₃ (368.48), MS (ESI): 369 (M + H⁺).

### (1 R,3S)-5-{3-[3-(5-Methyl-2-m-tolyl-oxazol-4-ylmethoxy)-cyclohexyloxy]-propyl}-2H-tetrazol

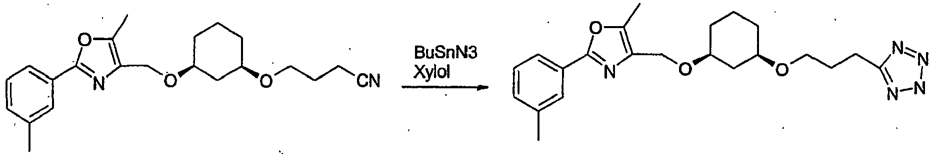

333 mg (1 R,3S)-4-[3-(5-Methyl-2-m-tolyl-oxazol-4-ylmethoxy)-cyclohexyloxy]-butyronitril werden in 5 ml Xylol gelöst und 919 mg Tributylzinnazid zugegeben. Man rührt 18 h bei 120°C und rührt nach dem Erkalten mit 0,5 ml TFA aus. Das Lösungsmittel wird im Vakuum entfernt und der Rückstand durch RP-HPLC gereinigt. Man erhält (1 R,3S)-5-{3-[3-(5-Methy)-2-m-tolyl-oxazol-4-ylmethoxy)-cyclohexyloxy]-propyl}-2H- tetrazol als farbloses Öl. C₂₂H₂₉N₅O₃ (411.51), MS (ESI): 412 (M + H⁺).

### Beispiel II

### (1 R,3S)-C,C,C-Trifluoro-N-{3-[3-(5-methyl-2-m-tolyl-oxazol-4-ylmethoxy)-cyclohexyl-oxy]-propyl}-methanesulfonamid

### 1 R,3S)-3-[3-(5-Methyl-2-m-tolyl-oxazol-4-ylmethoxy)-cyclohexyloxy]-propylamin

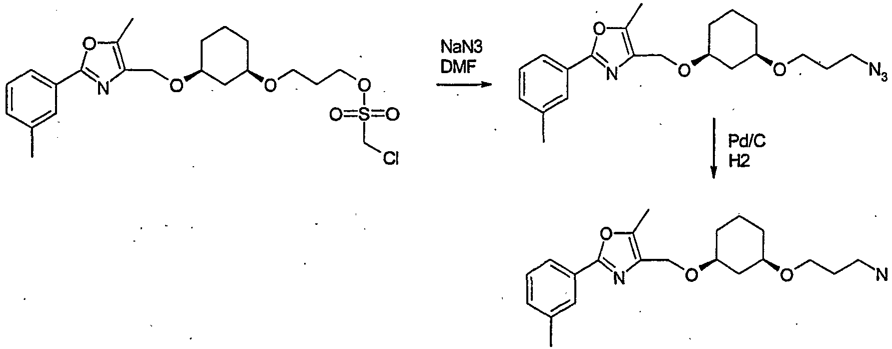

472 mg des 4-((1R,3S)-Chloro-methanesulfonsäure3-[3-(5-methy!-2-m-tolyl-oxazol-4-yl-methoxy)-cyclohexyloxy]-propylester werden in 3 ml N-Methylpyrrolidon gelöst und 200 mg Natriumazid zugegeben. Man rührt 3 h bei 60°C und nimmt nach dem Erkalten in 20 ml Ethylacetat/20 ml mit ges. NaCl-Lösung auf. Die organische Phase wird über Natriumsulfat getrocknet und das Lösungsmittel im Vakuum entfernt. Man erhält das 4-((1R,3S)-[3-(3-Azido-propoxy)-cyclohexyloxymethyl]-5-methyl-2-m-tolyl-oxazole als farbloses Öl. C₂₁H₂₈N₄O₃ (384.48), MS (ESI): 385 (M + H⁺).Das Rohazid wird in 20 ml Methanol aufgenommen und mit 50mg Pd/C 10% bei 1 bar Wasserstoffatmosphäre für 3 h hydriert. Man filtriert vom Katalysator und erhält (1 R,3S)-3-[3-(5-Methyl-2-m-tolyl-oxazol-4-ylmethoxy)-cyclohexyloxy]-propylamin als farbloses Öl. C₂₁H₃₀N₂O₃ (358.48), MS (ESI): 359 (M + H⁺).

### (1R,3S)-C,C,C-Trifluoro-N-{3-[3-(5-methyl-2-m-tolyl-oxazol-4-ylmethoxy)-cyclohexyl-oxy]-propyl}-methanesulfonamid

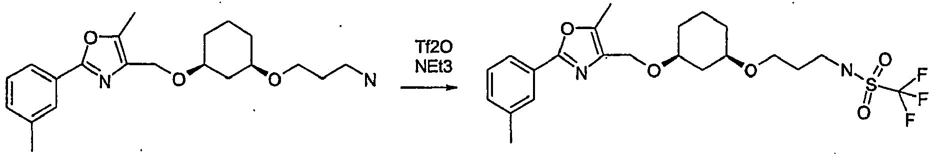

95 mg (1 R,3S)-3-[3-(5-Methyl-2-m-tolyl-oxazol-4-ylmethoxy)-cyclohexyloxy]-propylamin werden in 2 ml Dichlormethan gelöst 0,1 ml Triethylamin zugegeben und auf -78°C abgekühlt. Man gibt 60 µl Trifluormethansulfonsäureanhydrid zu und lässt langsam auf Raumtemp. kommen. Nach 3 h wurden alle flüchtigen Bestandteile im Vakuum entfernt und der Rückstand in DMF aufgenommen und filtriert. Die Verbindung wird durch RP-HPLC gereinigt. Man erhält (1 R,3S)-C,C,C-Trifluoro-N-{3-[3-(5-methyl-2-m-tolyl-oxazol-4-ylmethoxy)-cyclohexyl-oxy]-propyl}-methansulfonamid als farbloses Öl. C₂₂H₂₉F₃N₂O₅S (490.55), MS (ESI): 491 (M + H⁺).

### Beispiel III

### (1R,3S)-5-{3-[3-(5-Methyl-2-m-tolyl-oxazol-4-ylmethoxy)-cyclohexyloxy]-ethyl}-2H-tetrazol

### (1 R,3S)-4-[3-(5-Methyl-2-m-tolyl-oxazol-4-ylmethoxy)-cyclohexyloxy]-propionitril

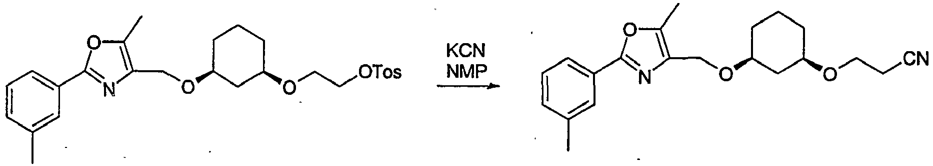

500 mg Toluol-4-sulfonsäure((1R,3S)-[3-(5-methyl-2-m-tolyl-oxazol-4-ylmethoxy)-cyclohexyloxy]-ethyl ester werden in 3 ml N-Methylpyrrolidon gelöst und 200 mg Kaliumcyanid sowie 30 mg Tetrabutylammoniumiodid zugegeben. Man rührt 8 h bei 50°C und nimmt nach dem Erkalten in 20 ml Ethylacetat/20 ml mit ges. NaCl-Lösung auf. Die organische Phase wird über Natriumsulfat getrocknet und das Lösungsmittel im Vakuum entfernt. Man erhält (1 R,3S)-4-[3-(5-Methyl-2-m-tolyl-oxazol-4-ylmethoxy)-cyclohexyloxy]-propionitril als farbloses Öl. C₂₂H₂₈N₂O₃ (368.48), MS (ESI): 369 (M + H⁺).

### (1R,3S)-5-{3-[3-(5-Methyl-2-m-tolyl-oxazol-4-ylmethoxy)-cyclohexyloxy]-ethyl}-2H-tetrazol

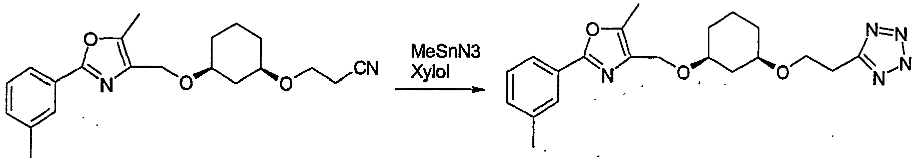

100 mg (1 R,3S)-4-[3-(5-Methyl-2-m-tolyl-oxazol-4-ylmethoxy)-cyclohexyloxy]-propionitril werden in 5 ml Xylol gelöst und 82 mg Trimethylzinnazid zugegeben. Man rührt 24 h bei 130°C und rührt nach dem Erkalten mit 0,5 ml TFA aus. Das Lösungsmittel wird im Vakuum entfernt und der Rückstand durch RP-HPLC gereinigt. Man erhält (1 R,3S)-5-{3-[3-(5-Methyl-2-m-tolyl-oxazol-4-ylmethoxy)-cyclohexyloxy]-ethyl}-2H- tetrazol als farbloses Öl. C₂₁H₂₇N₅O₃ (397.48), MS (ESI): 398 (M + H⁺).

### Beispiel IV

### (1 R,3S)-C,C,C-Trifluoro-N-{3-[3-(5-methyl-2-m-tolyl-oxazol-4-ylmethoxy)-cyclohexyl-oxy]-ethyl}-methanesulfonamid

### (1 R,3S)-3-[3-(5-Methyl-2-m-tolyl-oxazol-4-ylmethoxy)-cyclohexyloxy]-ethylamine

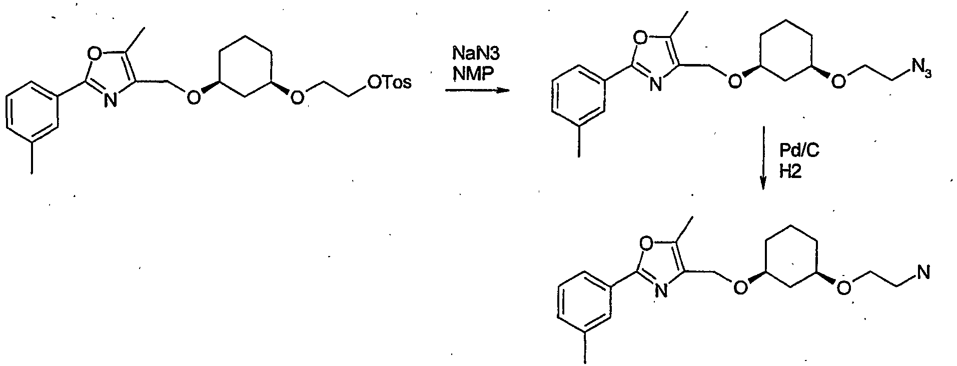

500 mg Toluol-4-sulfonsäure((1R,3S)-[3-(5-methyl-2-m-tolyl-oxazol-4-ylmethoxy)-cyclohexyloxy]-ethyl ester werden in 5 ml N-Methylpyrrolidon gelöst und 200 mg Natriumazid zugegeben. Man rührt 5 h bei 40°C und nimmt nach dem Erkalten in 20 ml Ethylacetat/20 ml mit ges. NaCl-Lösung auf. Die organische Phase wird über Natriumsulfat getrocknet und das Lösungsmittel im Vakuum entfernt. Man erhält das (1S,3R)-4-[3-(2-Azido-ethoxy)-cyclohexyloxymethyl]-5-methyl-2-m-tolyl-oxazol als farbloses Öl. C₂₀H₂₆N₄O₃ (370.46), MS (ESI): 371 (M + H⁺). Das Rohazid wird in 20 ml Methanol aufgenommen und mit 50 mg Pd/C 10% bei 1 bar Wasserstoffatmosphäre für 3 h hydriert. Man filtriert vom Katalysator und erhält (1 R,3S)-3-[3-(5-Methyl-2-m-tolyl-oxazo)-4-ylmethoxy)-cyclohexyloxy]-ethylamin als farbloses Öl. C₂₀H₂₈N₂O₃ (244.46), MS (ESI): 345 (M + H⁺).

### (1R,3S)-C,C,C-Trifluoro-N-(3-[3-(5-methyl-2-m-tolyl-oxazol-4-ylmethoxy)-cyclohexyl-oxy]-ethyl}-methanesulfonamid

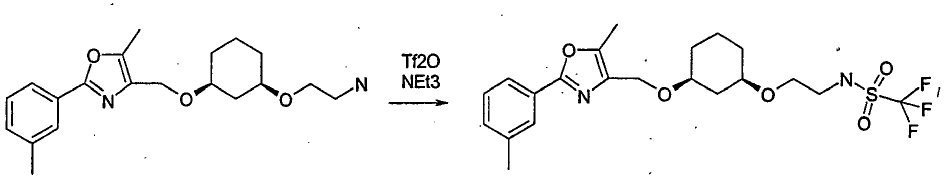

95 mg (1 R,3S)-3-[3-(5-Methy)-2-m-tolyl-oxazol-4-ylmethoxy)-cyclohexyloxy]-ethylamin werden in 2 ml Dichlormethan gelöst 0,1 ml Triethylamin zugegeben und auf-78°C abgekühlt. Man gibt 60 µl Trifluormethansulfonsäureanhydrid zu uns lässt langsam auf Raumtemp. kommen. Nach 3 h wurden alle flüchtigen Bestandteile im Vakkum entfernt und der Rückstand in DMF aufgenommen und filtriert. Die Verbindung wird durch RP-HPLC gereinigt. Man erhält (1 R,3S)-C,C,C-Trifluoro-N-{3-[3-(5-methyl-2-m-tolyl-oxazol-4-ylmethoxy)-cyclohexyl-oxy]-ethyl}-methanesulfonamid als farbloses Öl. C₂₁H₂₇F₃N₂O₅S (476.52), MS (ESI): 477 (M + H⁺).

### Beispiel V

### (1R,3S)-5,7-Difluoro-1-(2-[3-(5-methyl-2-m-tolyl-oxazol-4-ylmethoxy)-cyclohexyloxy]-ethyl}-1H- indole-2-carbonsäure

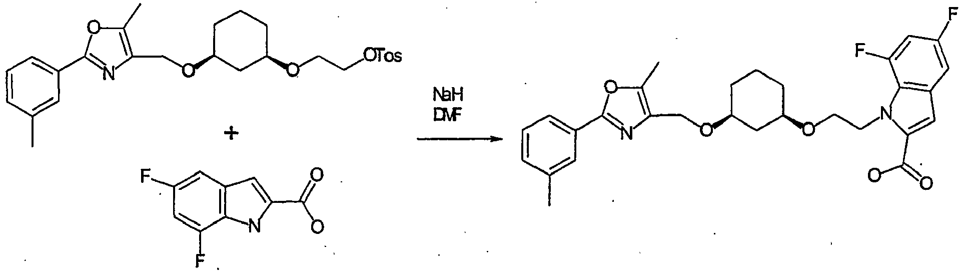

79 mg 5,7-Difluorindolcarbonsäure werden in 2 ml trockenem DMF gelöst und mit 34 mg 60-proz. Natriumhydrid-Suspension versetzt. Nach 30 min bei Raumtemp. werden 100 mg Toluol-4-sulfonsäure((1R,3S)-[3-(5-methyl-2-m-tolyl-oxazol-4-ylmethoxy)-cyclohexyloxy]-ethyl ester gelöst in 2 ml DMF zugegeben. Man rührt bei 65°C bis zum vollständigen Umsatz (LC-MS Kontrolle). Es werden 50 µl TFA zugegeben, filtriert und der Rückstand durch RP-HPLC gereinigt. Man erhält 40 mg (1R,3S)-5,7-Difluoro-1-{2-[3-(5-methyl-2-m-tolyl-oxazol-4-ylmethoxy)-cyclohexyloxy]-ethyl}-1H-indole-2-carbonsäure als farbloses Öl. C₂₉H₃₀F₂N₂O₅ (524.56), MS (ESI): 525 (M + H⁺).

### Beispiel VI

### (1 R,3S)-5-Benzyloxy-1-{2-[3-(5-methyl-2-m-tolyl-oxazol-4-ylmethoxy)-cyclohexyloxy]-ethyl}-1H- indol-2-carbonsäure

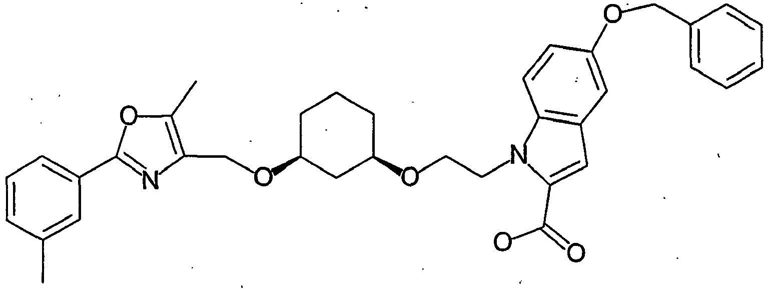

Aus Toluol-4-sulfonsäure-((1R,3S)-[3-(5-methyl-2-m-tolyl-oxazol-4-ylmethoxy)-cyclohexyloxy]- ethyl ester und 5-Benzyloxy-1 H-indol-2-carbonsäure erhält man analog zu Beispiel V (1 R,3S)-5-Benzyloxy-1-{2-[3-(5-methyl-2-m-tolyl-oxazol-4-ylmethoxy)-cyclohexyloxy]-ethyl}-1H-indol-2-carbonsäure mit dem Molekulargewicht 594,71 (C₃₆H₃₈N₂O₆), MS (ESI): 595 (M + H⁺).

### Beispiel VII

### (1 R,3S)-5-Ethyl-1-{2-[3-(5-methyl-2-m-tolyl-oxazol-4-ylmethoxy)-cyclohexyloxy]-ethyl}-1 H- indol-2-carbonsäure

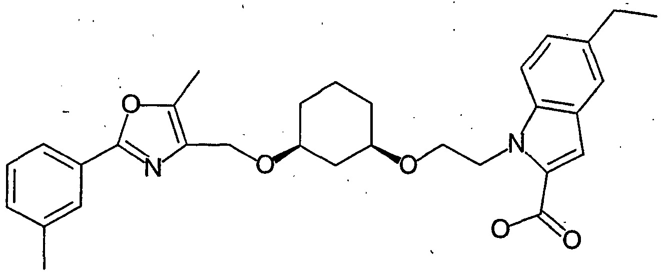

Aus Toluol-4-sulfonsäure-((1R,3S)-[3-(5-methyl-2-m-tolyl-oxazol-4-ylmethoxy)-cyclohexyloxy]- ethyl ester und 5-Ethyl-1 H-indol-2-carbonsäure erhält man analog zu Beispiel V (1 R,3S)-5-Ethyl-1-{2-[3-(5-methyl-2-m-tolyl-oxazol-4-ylmethoxy)-cyclohexyloxy]-ethyl}-1H- indol-2-carbonsäure mit dem Molekulargewicht 516.64 (C₃₁H₃₆N₂O₅), MS (ESI): 517 (M + H⁺).

### Beispiel VIII

### (1R,3S)-5-Bromo-1-{2-[3-(5-methyl-2-m-tolyl-oxazol-4-ylmethoxy)-cyclohexyloxy]-ethyl}-1H- indol-2-carbonsäure

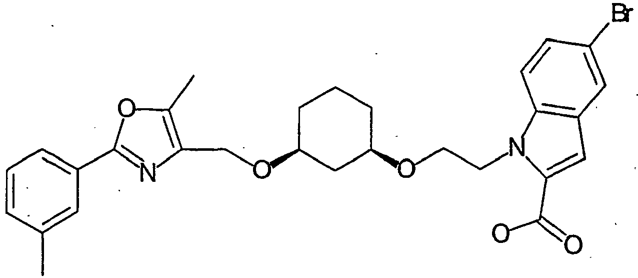

Aus Toluol-4-sulfonsäure-((1R,3S)-[3-(5-methyl-2-m-tolyl-oxazol-4-ylmethoxy)-cyclohexyloxy]- ethyl ester und 5-Bromo-1H-indol-2-carbonsäure erhält man analog zu Beispiel I (1R,3S)-5-Bromo-1-{2-[3-(5-methyl-2-m-tolyl-oxazol-4-ylmethoxy)-cyclohexyloxy]-ethyl}-1H-indol-2-carbonsäure mit dem Molekulargewicht 567,48 (C₂₉H₃₁BrN₂O₅), MS (ESI): 568 (M + H⁺).

### Beispiel IX

### (1 R,3S)-5-(2-Methoxy-ethoxy)-1-{2-[3-(5-methyl-2-m-tolyl-oxazol-4-ylmethoxy)-cyclohexyloxy]- ethyl}-1H-pyrrolo[2,3-c]pyridin-2-carbonsäure

### 2-(2-Methoxy-ethoxy)-4-methyl-5-nitro-pyridin

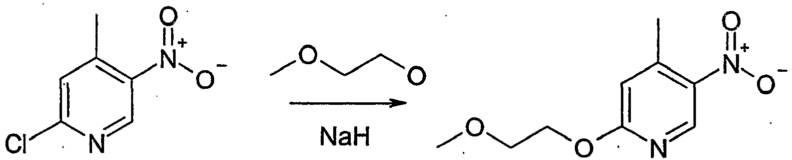

In 10 ml Etyhlenglycolmonomethylether wurden 60-proz. Natriumhydrid-Suspension eingetragen und bis zum Ende der Wasserstoffentwicklung gerührt. Man trägt 516 mg 2-Chloro-4-methyl-5-nitro-pyridin ein und rührt bis zum vollständigen Umsatz bei Raumtemperatur. Man addiert 20 ml Wasser und extrahiert mit Methyl-tert.-butylether. Die organische Phase wird über Natriumsulfat getrocknet und das Lösungsmittel im Vakuum entfernt. Der Rückstand wird durch Flash-Chromatographie an Kieselgel (n-Heptan/Ethylacetat = 1:3) gereinigt. Man erhält 2-(2-Methoxy-ethoxy)-4-methyl-5-nitro-pyridin als farbloses Öl. C₉H₁₂N₂O₄ (212.21), MS (ESI): 213 (M + H⁺).

### 3-[2-(2-Methoxy-ethoxy)-5-nitro-pyridin-4-yl]-2-oxo-propionsäureethylester-Kaliumsalz

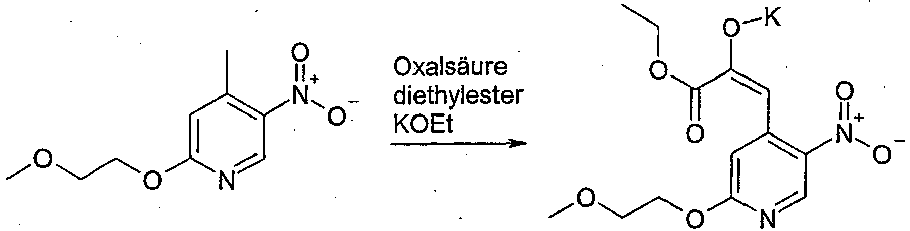

1,86 g Kalium werden in 120 ml trockenem Diethylther vorgelegt und 15 ml EtOH langsam zugegeben. Man kühlt auf 0°C. 5 g 2-(2-Methoxy-ethoxy)-4-methyl-5-nitro-pyridin werden in 15 ml trockenem Ether und 3 ml EtOH gelöst und zugegeben. In 100 ml Toluol wurden 27,5 g, Oxalsäurediethylester gelöst und bei 0°C in 45 min zugetropft. Man rührt 24 h bei Raumtemp. Man lässt den Niederschlag absitzen, filtriert und wäscht den Niederschlag mit Ether/n-Heptan 1:1. Der Niederschlag wird in Hochvakuum getrocknet. Man erhält 3-[2-(2-Methoxy-ethoxy)-5-nitro-pyridin-4-yl]-2-oxo-propionsäureethylester-Kaliumsalz als roten Feststoff. (LC-MS protonierte Form) C₁₃H₁₆N₂O₇ (312.28), MS (ESI): 313 (M + H⁺).

### 5-(2-Methoxy-ethoxy)-1H-pyrrolo[2,3-c]pyridin-2-carbonsäureethyl ester

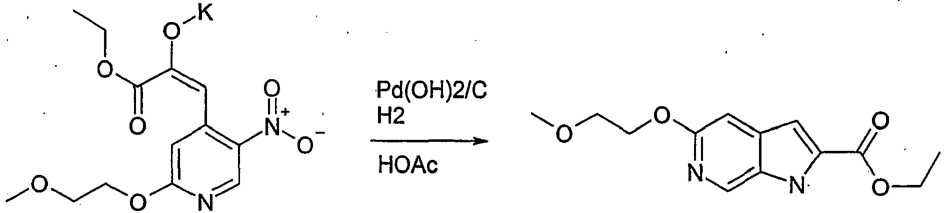

3,5 g getrocknetes 3-[2-(2-Methoxy-ethoxy)-5-nitro-pyridin-4-yl]-2-oxo-propionsäureethylester-Kaliumsalz werden in 50 ml MeOH gelöst und mit 2 ml Essigsäure versetzt. Nach Zugabe von 500 mg Palladiumhydroxid/C 20% wird unter 1 atm Wasserstoff gerührt. Nach 5 h erfolgt die Zugabe von weiteren 200 mg Katalysator und 0,75 ml Trifluorethanol. Nach weiteren 3 h werden alle flüchtigen Bestandteile im Vakuum entfernt und 20 ml ges Natriumhydrogencarbonat-Lösung addiert und Ethylacetat extrahiert. Die organische Phase wird über Natriumsulfat getrocknet und das Lösungsmittel im Vakuum entfernt. Der Rückstand wird durch Flash-Chromatographie an Kieselgel (n-Heptan/Ethylacetat = 1:2) gereinigt. Man erhält 5-(2-Methoxy-ethoxy)-1H-pyrrolo[2,3-c]pyridin-2-carbonsäureethyl ester als gelblichen Feststoff. C₁₃H₁₆N₂O₄ (264.28), MS (ESI): 265 (M + H⁺).

### 5-(2-Methoxy-ethoxy)-1-{2-[3-(5-methyl-2-m-tolyl-oxazol-4-ylmethoxy)-cyclohexyloxy]-ethyl}-1H-pyrrolo[2,3-c]pyridin-2-carbonsäureethyl ester

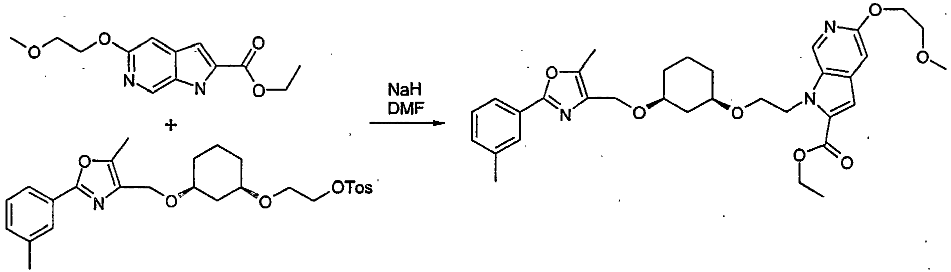

66 mg 5-(2-Methoxy-ethoxy)-1H-pyrrolo[2,3-c]pyridin-2-carbonsäureethyl ester werden in 2 ml trockenem DMF gelöst und mit 12 mg 60-proz. Natriumhydrid-Suspension versetzt. Nach 30 min bei Raumtemp. werden 100 mg des Toluol-4-sulfonsäure((1R,3S)-[3-(5-methyl-2-m-tolyl-oxazol-4-ylmethoxy)-cyclohexyloxy]-ethyl ester Toluol-4-sulfonsäure((1R,3S)-[3-(5-methyl-2-m-tolyl-oxazol-4-ylmethoxy)-cyclohexyloxy]-ethyl ester in 2 ml DMF zugegeben. Man rührt bei 40°C bis zum vollständigen Umsatz (LC-MS Kontrolle). Nach dem Erkalten wird mit ges. NaCl-Lösung und Ethylacetat verdünnt. Die organische Phase wird über Natriumsulfat getrocknet und das Lösungsmittel im Vakuum entfernt. Der Rückstand wird durch Flash-Chromatographie an Kieselgel (n-Heptan/Ethylacetat = 1:2) gereinigt. Man erhält 5-(2-Methoxy-ethoxy)-1-{2-[3-(5-methyl-2-m-tolyl-oxazol-4-ylmethoxy)-cyclo-hexyloxy]-ethyl}-1H-pyrrolo[2,3-c]pyridin-2-carbonsäureethylester als gelblichen Feststoff. C₃₃H₄₁N₃O₇ (591.71), MS (ESI): 592 (M + H⁺).

### (1 R,3S)-5-(2-Methoxy-ethoxy)-1-{2-[3-(5-methyl-2-m-tolyl-oxazol-4-ylmethoxy)-cyclohexyloxy]- ethyl}-1H-pyrrolo[2,3-c]pyridin-2-carbonsäure

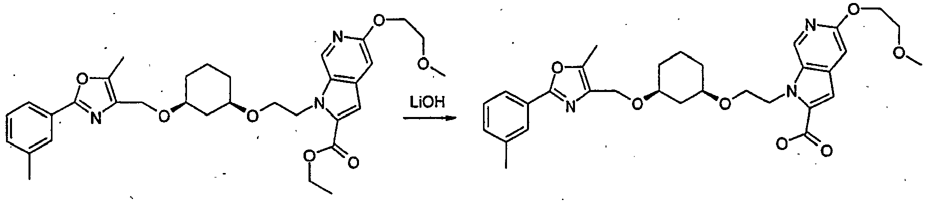

60 mg 5-(2-Methoxy-ethoxy)-1-{2-[3-(5-methyl-2-m-tolyl-oxazol-4-ylmethoxy)-cyclohexyloxy]-ethyl}-1H-pyrrolo[2,3-c]pyridin-2-carbonsäureethylester werden in 2 ml THF/Methanol 3:1 gelöst und mit 0,15 ml einer 1 N Lithiumhydroxid-Lösung versetzt. Man rührt 24 h bei Raumtemp. Alle flüchtigen Bestandteile werden im Vakuum entfernt, der Rückstand in DMF/Acetontril aufgenommen, 70 µl TFA zugegeben, filtriert und das Gemisch durch RP-HPLC gereinigt. Man erhält 14 mg (1 R,3S)-5-(2-Methoxy-ethoxy)-1-{2-[3-(5-methyl-2-m-tolyl-oxazol-4-ylmethoxy)-cyclo-hexyloxy]-ethyl}-1H-pyrrolo[2,3-c]pyridin-2-carbonsäure als farbloses Öl. C₃₁H₃₇N₃O₇ (563.66), MS (ESI): 564 (M + H⁺).

### Beispiel X

### (1 R,3S)-5-Cyano-1-{2-[3-(5-methyl-2-m-tolyl-oxazol-4-ylmethoxy)-cyclohexyloxy]-ethyl}-1H- indol-2-carbonsäure

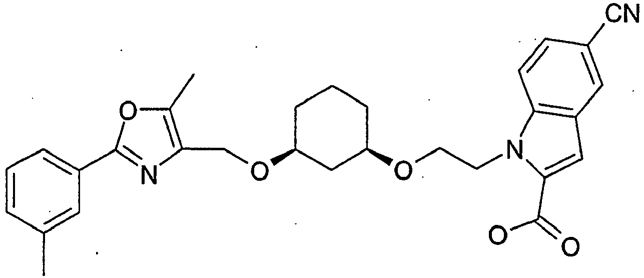

Aus Toluol-4-sulfonsäure-((1R,3S)-[3-(5-methyl-2-m-tolyl-oxazol-4-ylmethoxy)-cyclo-hexyloxy]- ethyl ester und 5-Cyano-1H-indol-2-carbonsäureethylester erhält man analog zu Beispiel IX das (1 R,3S)-5-Cyano-1-{2-[3-(5-methyl-2-m-tolyl-oxazol-4-ylmethoxy)-cyclohexyloxy]-ethyl}-1H-indol-2-carbonsäure mit dem Molekulargewicht 513.60 (C₃₀H₃₁N₃O₅), MS (ESI): 514 (M + H⁺).

### Beispiel XI

### (1R,3S)-6-Methoxy-1-{2-[3-(5-methyl-2-m-tolyl-oxazol-4-ylmethoxy)-cyclohexyloxy]-ethyl}-1H- indol-2-carbonsäure

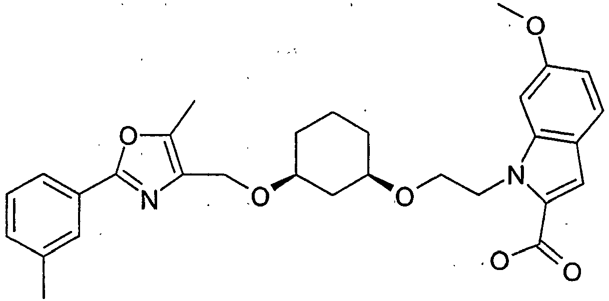

Aus Toluol-4-sulfonsäure-((1R,3S)-[3-(5-methyl-2-m-tolyl-oxazol-4-ylmethoxy)-cyclohexyloxy]-ethyl ester und 6-Methoxy-1H-indol-2-carbonsäuremethylester erhält man analog zu Beispiel IX (1R,3S)-6-Methoxy-1-{2-[3-(5-methyl-2-m-tolyl-oxazol-4-ylmethoxy)-cyclohexyloxy]-ethyl}-1H-indol-2-carbonsäure mit dem Molekulargewicht 518.62 (C₃₀H₃₄N₂O₆), MS (ESI): 519 (M + H⁺).

### Beispiel XII

### (1R,3S)-5,6-Dimethoxy-1-{2-[3-(5-methyl-2-m-tolyl-oxazol-4-ylmethoxy)-cyclohexyloxy]-ethyl}-1H- indole-2-carbonsäure

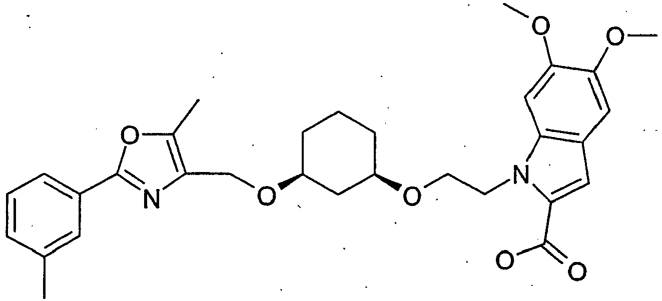

Aus Toluol-4-sulfonsäure-((1R,3S)-[3-(5-methyl-2-m-tolyl-oxazol-4-ylmethoxy)-cyclohexyloxy]- ethyl ester und 5,6-Dimethoxy-1H-indol-2-carbonsäureethylester erhält man analog zu Beispiel IX (1R,3S)-5,6-Dimethoxy-1-{2-[3-(5-methyl-2-m-tolyl-oxazol-4-ylmethoxy)-cyclohexyloxy]-ethyl}-1H-indol-2-carbonsäure mit dem Molekulargewicht 548.64 (C₃₁H₃₆N₂O₇), MS (ESI): 549 (M + H⁺).

### Beispiel XIII

### (1R,3S)-5-Methanesulfonyl-1-{2-[3-(5-methyl-2-m-tolyl-oxazol-4-ylmethoxy)-cyclohexyloxy]-ethyl}-1 H-indole-2-carbonsäure

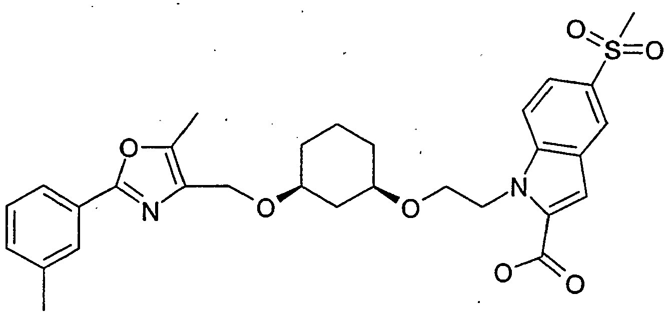

Aus Toluol-4-sulfonsäure-((1R,3S)-[3-(5-methyl-2-m-tolyl-oxazol-4-ylmethoxy)-cyclohexyloxy]-ethylester und 5-Methanesulfonyl-1 H-indol-2-carbonsäure-methylester erhält man analog zu Beispiel IX (1 R,3S)-5-Methanesulfonyl-1-{2-[3-(5-methyl-2-m-tolyl-oxazol-4-ylmethoxy)-cyclohexyloxy]-ethyl}-1H-indole-2-carbonsäure mit dem Molekulargewicht 566.68 (C₃₀H₃₄N₂O₇S), MS (ESI): 567 (M + H⁺).

### Beispiel XIV

### (1R,3S)-2-Methyl-6-{2-[3-(5-methyl-2-m-tolyl-oxazol-4-ylmethoxy)-cyclohexyloxy]-ethoxy}-benzoesäure

### (1R,3S)-2-Methyl-6-{2-[3-(5-methyl-2-m-tolyl-oxazol-4-ylmethoxy}-cyclohexyloxy]-ethoxy}-benzoesäureethyl ester

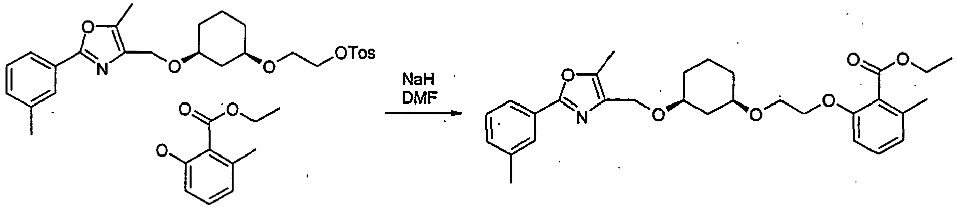

90 mg 2-Hydroxy-6-methyl-benzoesäureethylester werden in 2 ml trockenem DMF gelöst und mit 23 mg 60-proz. Natriumhydrid-Suspension versetzt. Nach 30 min bei Raumtemp. werden 100 mg Toluol-4-sulfonsäure-((1R,3S)-[3-(5-methyl-2-m-tolyl-oxazol-4-ylmethoxy)-cyclohexyloxy]-ethylester in 2 ml DMF zugegeben. Man rührt bei 40°C bis zum vollständigen Umsatz (LC-MS Kontrolle). Nach dem Erkalten wird mit ges. NaCl-Lösung und Ethylacetat verdünnt. Die organische Phase wird über Natriumsulfat getrocknet und das Lösungsmittel im Vakuum entfernt. Man erhält (1 R,3S)-2-Methyl-6-{2-[3-(5-methyl-2-m-tolyl-oxazol-4-ylmethoxy)-cyclohexyloxy]-ethoxy}-benzoesäureethyl ester als gelbliches Öl. C₃₀H₃₇NO₆ (507.63), MS (ESI): 508 (M + H⁺).

### (1R,3S)-2-Methyl-6-{2-[3-(5-methyl-2-m-tolyl-oxazol-4-ylmethoxy)-cyclohexyloxy]-ethoxy}-benzoesäure

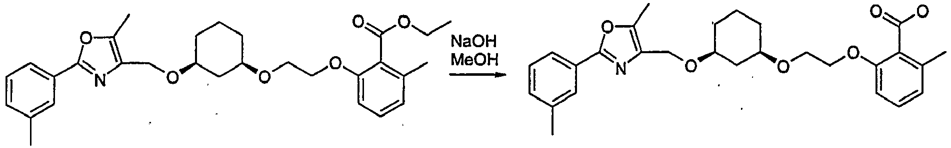

Ungereinigter (1R,3S)-2-Methyl-6-{2-[3-(5-methyl-2-m-tolyl-oxazol-4-ylmethoxy)-cyclohexyloxy]-ethoxy}-benzoesäureethyl ester werden in 3 ml Methanol gelöst und mit 0,25 ml einer 5 N Natriumhydroxid-Lösung versetzt. Man rührt 8 h bei 60°C und 12 h bei 80°C. Nach dem Erkalten wird in 2N HCl/Ethylacetat aufgenommen, die organische Phase über Natriumsulfat getrocknet und das Lösungsmittel im Vakuum entfernt. Der Rückstand wird durch RP-HPLC gereinigt. Man erhält (1 R,3S)-2-Methyl-6-{2-[3-(5-methyl-2-m-tolyl-oxazol-4-ylmethoxy)-cyclohexyloxy]-ethoxy}-benzoesäure als farbloses Öl. C₂₈H₃₃NO₆ (479.58), MS (ESI): 480 (M + H⁺).

### Beispiel XV

### (1R,3S)-2-{2-[3-(5-methyl-2-m-tolyl-oxazol-4-ylmethoxy)-cyclohexyloxy]-ethoxy}-benzoesäure

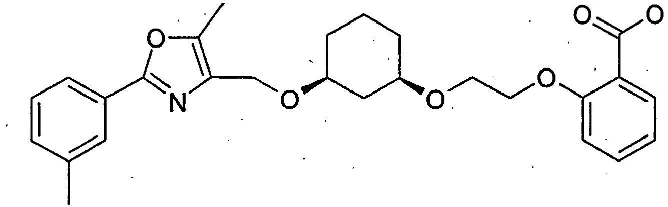

Aus Toluol-4-sulfonsäure-((1R,3S)-[3-(5-methyl-2-m-tolyl-oxazol-4-ylmethoxy)-cyclohexyloxy]- ethyl ester und 2-Hydroxybenzoesäuremethylester erhält man analog zu Beispiel XIV (1R,3S}-2-{2-[3-(5-methyl-2-m-tolyl-oxazol-4-ylmethoxy)-cyclohexyloxy]-ethoxy}-benzoesäure mit dem Molekulargewicht 465.55 (C₂₇H₃₁NO₆), MS (ESI): 466 (M + H⁺).

### Beispiel XVI

### (1R,3S)-2-Fluoro-6-{2-[3-(5-methyl-2-m-tolyl-oxazol-4-ylmethoxy)-cyclohexyloxy]-ethoxy}-benzoesäure

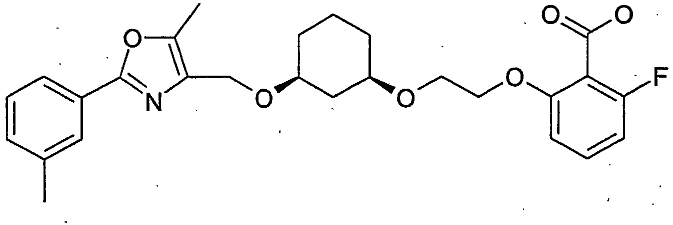

Aus Toluol-4-sulfonsäure-((1R,3S)-[3-(5-methyl-2-m-tolyl-oxazol-4-ylmethoxy)-cyclo-hexyloxy]-ethyl ester und 2-Fluoro-6-hydroxy-benzoesäuremethylester erhält man analog zu Beispiel XIV (1R,3S)-2-Fluoro-6-{2-[3-(5-methyl-2-m-tolyl-oxazol-4-ylmethoxy)-cyclohexyloxy]-ethoxy}-benzoesäure mit dem Molekulargewicht 483.54 (C₂₇H₃₀FNO₆), MS (ESI): 484 (M + H⁺).

### Beispiel XVII

### (1R,3S)-2-Fluoro-6-{2-[3-(5-methyl-2-m-tolyl-oxazol-4-ylmethoxy)-cyclohexyloxy]-ethoxy}-benzoesäure

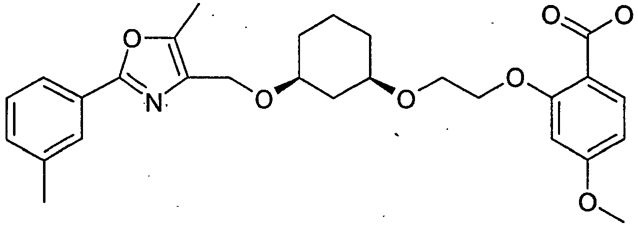

Aus Toluene-4-sulfonsäure ((1R,3S)-[3-(5-methyl-2-m-tolyl-oxazol-4-ylmethoxy)-cyclohexyloxy]-ethyl ester und 4-Methoxy-2-hydroxy-benzoesäuremethylester erhält man analog zu Beispiel XIV (1 R,3S)-2-Fluoro-6-{2-[3-(5-methyl-2-m-tolyl-oxazol-4-ylmethoxy)-cyclohexyloxy]-ethoxy}-benzoesäure mit dem Molekulargewicht 495.58 (C₂₈H₃₃NO₇), MS (ESI): 496 (M + H⁺).

### Beispiel XVIII

### (1R,3S)-4-Isobutoxy-2-{2-[3-(5-methyl-2-m-tolyl-oxazol-4-ylmethoxy)-cyclohexyloxy]-ethoxy}-benzoesäure

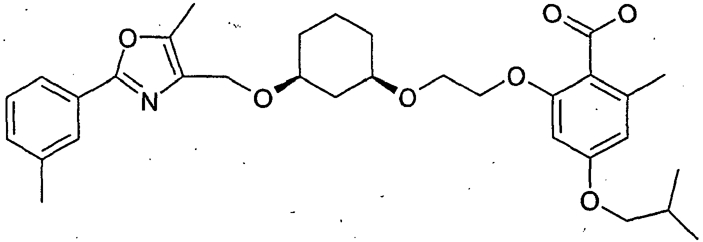

Aus Toluene-4-sulfonsäure ((1R,3S)-[3-(5-methyl-2-m-tolyl-oxazol-4-ylmethoxy)-cyclohexyloxy]-ethyl ester und 4-Isoprpoxy-2-hydroxy-6-methyl-benzoesäure-ethylester erhält man analog zu Beispiel XIV (1R,3S)-4-Isobutoxy-2-{2-[3-(5-methyl-2-m-tolyl-oxazol-4-ylmethoxy)-cyclohexyloxy]-ethoxy}-benzoesäure mit dem Molekulargewicht 551.69 (C₃₂H₄₁NO₇), MS (ESI): 552 (M + H⁺).

### Beispiel XIX

### (1R,3S)-4-Benzyloxy-2-{2-[3-(5-methyl-2-m-tolyl-oxazol-4-ylmethoxy)cyclohexyloxy]-ethoxy}- benzoesäure

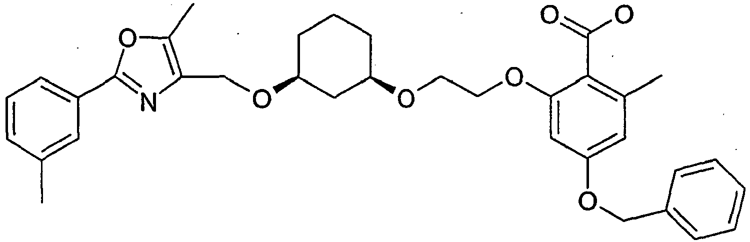

Aus Toluene-4-sulfonsäure ((1R,3S)-[3-(5-methyl-2-m-tolyl-oxazol-4-ylmethoxy)-cyclohexyloxy]-ethyl ester und 4-Benzyloxy-2-hydroxy-6-methyl-benzoesäureethylester erhält man analog zu Beispiel XIV (1R,3S)-4-Benzyloxy-2-{2-[3-(5-methyl-2-m-tolyl-oxazol-4-ylmethoxy)-cyclohexyloxy]-ethoxy}- benzoesäure mit dem Molekulargewicht 585.70 (C₃₅H₃₉NO₇), MS (ESI): 586 (M + H⁺).

### Beispiel XX

### (1S,3R)-5-Methyl-4-{3-[2-(2-nitro-phenoxy)-ethoxy]-cyclohexyloxymethyl}-2-m-tolyl-oxazol

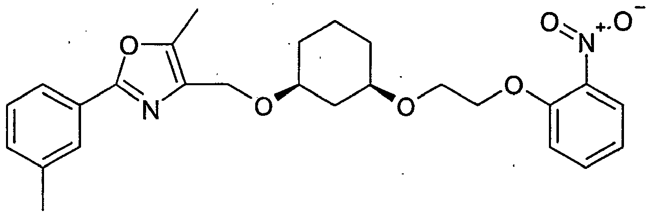

Aus Toluene-4-sulfonsäure ((1R,3S)-[3-(5-methyl-2-m-tolyl-oxazol-4-ylmethoxy)-cyclohexyloxy]-ethyl ester und 2-Hydroxynitrobenzol erhält man analog zu Beispiel XIV (1S,3R)-5-Methyl-4-{3-[2-(2-nitro-phenoxy)-ethoxy]-cyclohexyloxymethyl}-2-m-tolyl-oxazol mit dem Molekulargewicht 466.54 (C₂₆H₃₀N₂O₆), MS (ESI): 467 (M + H⁺).

### Beispiel XXI

### (1S,3R)-5-Methyl-4-{3-[2-(3-methyl-2-nitro-phenoxy)-ethoxy]-cyclohexyloxymethyl}-2-m-tolyl- oxazol

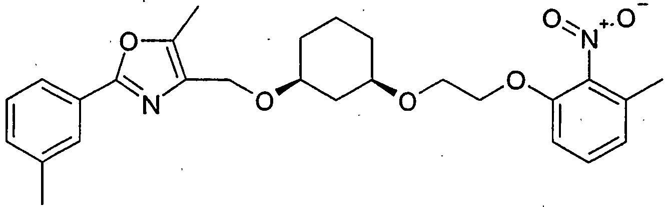

Aus Toluene-4-sulfonsäure ((1R,3S)-[3-(5-methyl-2-m-tolyl-oxazol-4-ylmethoxy)-cyclohexyloxy]-ethyl ester und 6-Methyl-2-hydroxynitrobenzol erhält man analog zu Beispiel XIV (1S,3R)-5-Methyl-4-{3-[2-(3-methyl-2-nitro-phenoxy)-ethoxy]-cyclohexyl-oxymethyl}-2-m-tolyl-oxazol mit dem Molekulargewicht 480.57 (C₂₇H₃₂N₂O₆), MS (ESI): 481 (M + H⁺).

### Beispiel XXII

### (1R,3S)-2-{2-[3-(5-methyl-2-m-tolyl-oxazol-4-ylmethoxy)-cyclohexyloxy]-ethoxy}-phenylboronsäure

### (1S,3R)-5-Methyl-4-(3-{2-[2-(4,4,5,5-tetramethyl-[1,3,2]dioxaborolan-2-yl)-phenoxy]-ethoxy}-cyclohexyloxymethyl)-2-m-tolyl-oxazol

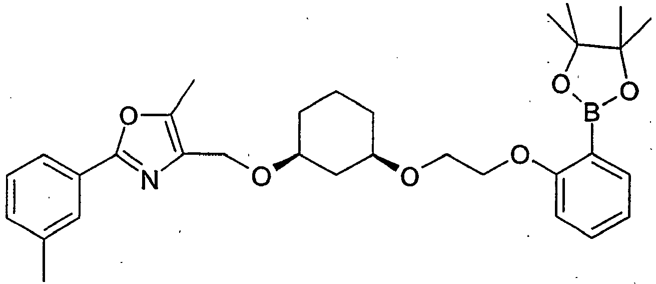

Aus Toluene-4-sulfonsäure ((1R,3S)-(3-(5-methyl-2-m-tolyl-oxazol-4-ylmethoxy)-cyclohexyloxy]-ethyl ester und 2-(4,4,5,5-Tetramethy)-[1,3,2]dioxaboro)an-2-yl)-phenol erhält man analog zu Beispiel XIV (1S,3R)-5-Methyl-4-(3-{2-[2-(4,4,5,5-tetramethyl-[1,3,2]dioxaborolan-2-yl)-phenoxy]-ethoxy}-cyclohexyloxymethyl)-2-m-tolyl-oxazol mit dem Molekulargewicht 547.51 (C₃₂H₄₂BNO₆), MS (ESI): 548 (M + H⁺).

### (1R,3S)-2-{2-[3-(5-methyl-2-m-tolyl-oxazol-4-ylmethoxy)-cyclohexyloxy]-ethoxy}-phenylboronsäure

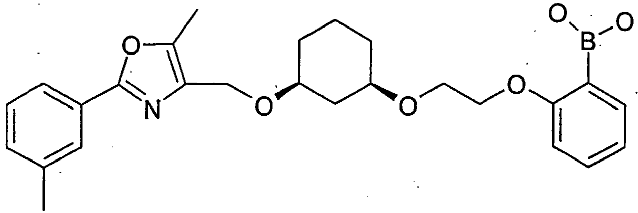

Ungereinigtes (1S,3R)-5-Methyl-4-(3-{2-[2-(4,4,5,5-tetramethyl-[1,3,2]dioxa-borolan-2-yl)-phenoxy]-ethoxy}-cyclohexyloxymethyl)-2-m-tolyl-oxazol wird in 6 ml THF aufgenommen und mit 0.75 ml 1N HCl versetzt. Nach 3 h rühren bei Raumtemp wird evaporiert und der Rückstand in DMF aufgenommen und durch RP-HPLC gereinigt. Man erhält (1R,3S)-2-{2-[3-(5-methyl-2-m-tolyl-oxazol-4-ylmethoxy)-cyclohexyloxy]-ethoxy}-phenylboronsäure als farbloses Öl. 465.36 (C₂₆H₃₂BNO₆), MS (ESI): 466 (M + H⁺).

### Beispiel XXIII

### (1R,3S)-3-{2-[3-(5-Methyl-2-m-tolyl-oxazol-4-ylmethoxy)-cyclohexyloxy]-ethoxy}-5-trifluoromethyl-thiophene-2-carbonsäure

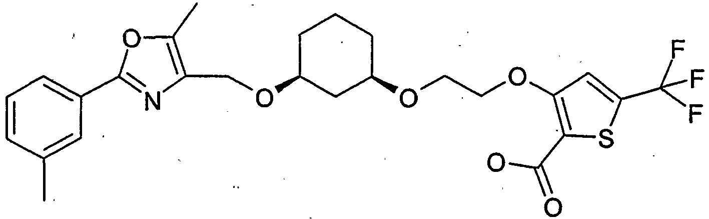

Aus Toluene-4-sulfonsäure ((1R,3S)-[3-(5-methyl-2-m-tolyl-oxazol-4-ylmethoxy)-cyclohexyloxy]-ethyl ester und 3-Hydroxy-5-trifluoromethyl-thiophene-2-carbonsäure-methylester erhält man analog zu Beispiel XIV (1R,3S)-3-{2-[3-(5-Methyl-2-m-tolyl-oxazol-4-ylmethoxy)-cyclohexyloxy]-ethoxy}-5-trifluoromethyl-thiophene-2-carbonsäure mit dem Molekulargewicht 539.58 (C₂₆H₂₈F₃NO₆S), MS (ESI): 540 (M + H⁺).

### Beispiel XXIV

### (1R,3S)-C,C,C-Trifluoro-N-{3-[3-(5-methyl-2-m-tolyl-oxazol-4-ylmethoxy)-cyclohexyloxy methyl]-phenyl}-methansulfonamid

### (1S,3R)-5-Methyl-4-[3-(3-nitro-benzyloxy)-cyclohexyloxymethyl]-2-m-tolyl-oxazol

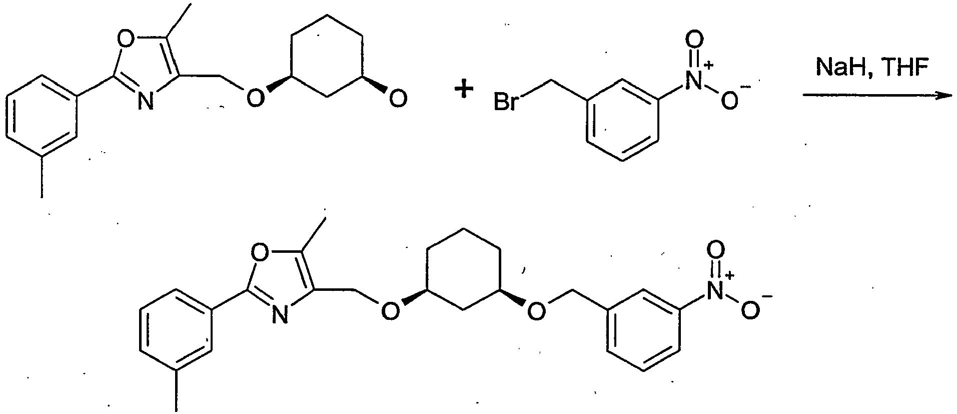

Zu 150 mg 3-((1R,3S)-cis-5-Methyl-2-m-tolyl-oxazol-4-ylmethoxy)-cyclohexanol in 3 ml THF gibt man 530 µl 1M NaHMDS in THF und rührt für 10 min bei RT. 121 mg 3-Nitrobenzylbromid werden zugegeben und bei 60°C für 5 h gerührt. Nach Zugabe von weiterem 3-Nitrobenzylbromid wird über Nacht gerührt. Die Mischung wird in ges. NaCl-Lösung/Essigester aufgenommen. Man trocknet die org. Phase über Natriumsulfat und evaporiert. Der Rückstand wird durch Chromatographie an Kieselgel gereinigt. Man erhält (1S,3R)-5-Methyl-4-[3-(3-nitro-benzyloxy)-cyclohexyl-oxymethyl]-2-m-tolyl-oxazol als farbloses Öl mit dem Molekulargewicht 436.51 (C₂₅H₂₈N₂O₅), MS (ESI): 437 (M + H⁺).

### (1R,3S)-3-[3-(5-Methyl-2-m-tolyl-oxazol-4-ylmethoxy)-cyclohexyloxymethyl]-phenylamin

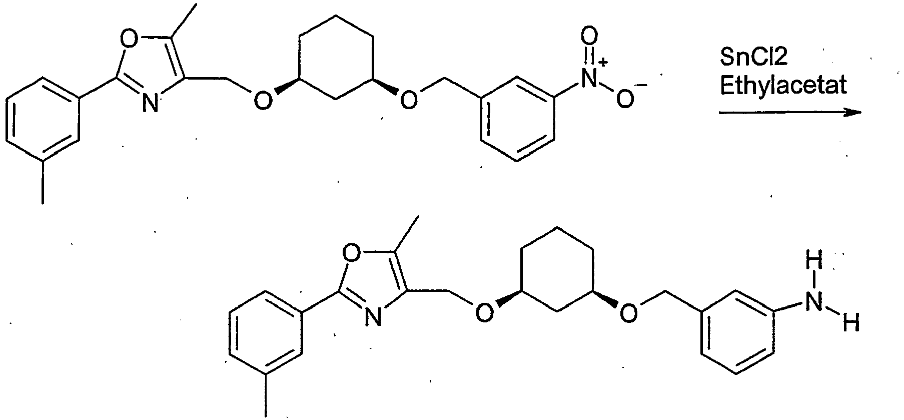

Zu 150 mg (1S,3R)-5-Methyl-4-[3-(3-nitro-benzyloxy)-cyclohexyloxymethyl]-2-m-tolyl-oxazol in 24 ml Essigester gibt man 400 mg Zinn(II)chlorid dihydrat und rührt bei RT. Nach jeweils 8 h werden 400 mg Zinn(II)chlorid dihydrat zugesetzt bis vollständiger Umsatz erreicht ist. Man verdünnt die Mischung mit Essigester und wäscht mit Wasser. Man trocknet die org. Phase über Natriumsulfat und evaporiert. Der Rückstand wird ohne Reinigung in die nächste Reaktion eingesetzt. Man erhält (1R,3S)-3-[3-(5-Methyl-2-m-tolyl-oxazol-4-ylmethoxy)-cyclohexyloxymethyl]-phenylamin als farbloses Öl mit dem Molekulargewicht 406.53 (C₂₅H₃₀N₂O₃), MS (ESI): 407 (M + H⁺).

### (1R,3S)-C,C,C-Trifluoro-N-{3-[3-(5-methyl-2-m-tolyl-oxazol-4-ylmethoxy)-cyclohexyloxy methyl]-phenyl}-methansulfonamid

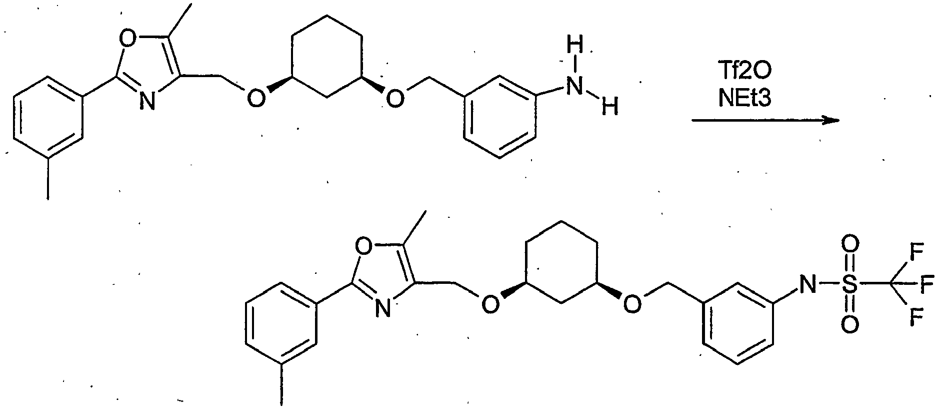

55 mg (1R,3S)-3-[3-(5-Methyl-2-m-tolyl-oxazol-4-ylmethoxy)-cyclohexyloxymethyl]-phenylamin werden inn2 ml Dichlormethan gelöst und auf -78°C abgekühlt. Man gibt 100 µl Triethylamin gefolgt von 42 µl Trifluormethansulfonsäureanhydrid zu. Nach 1 h werden 200 µl Wasser zugegeben und lässt auf Zimmertemperatur erwärmen. Man verdünnt mit Dichlormethan, wäscht mit 1 n HCL und ges. NaCl-Lösung. Man trocknet die org. Phase über Natriumsulfat und evaporiert. Der Rückstand wird RP-HPLC gereinigt. Man erhält (1R,3S)-C,C,C-Trifluoro-N-{3-[3-(5-methyl-2-m-tolyl-oxazol-4-ylmethoxy)-cyclohexyloxymethyl]-phenyl}-methansulfonamid als farbloses Öl mit dem Molekulargewicht 538.59 (C₂₆H₂₉F₃N₂O₅S), MS (ESI): 539 (M + H⁺).

### Beispiel XXV

### (1R,3S)-2-[3-(5-Methyl-2-m-tolyl-oxazol-4-ylmethoxy)-cyclohexyloxymethyl]-benzolboronsäure

### (1S,3R)-5-Methyl-4-{3-[2-(4,4,5,5-tetramethyl-[1,3,2]dioxaborolan-2-yl)-benzyloxy]-cyclohexyloxymethyl}-2-m-tolyl-oxazole

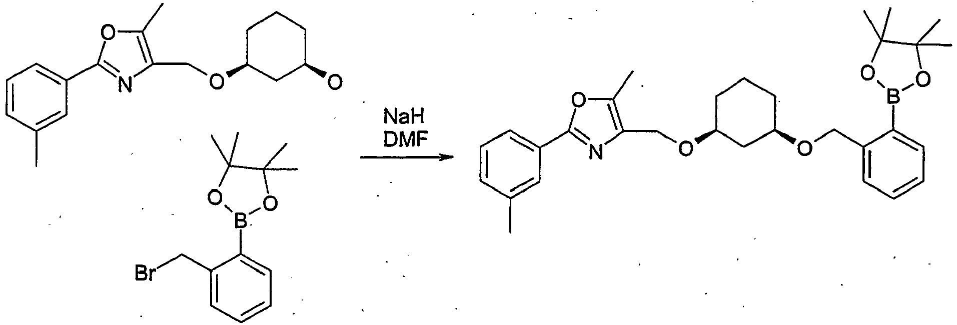

Zu 300 mg 3-((1R,3S)-cis-5-Methyl-2-m-tolyl-oxazol-4-ylmethoxy)-cyclohexanol in 5 ml DMF gab man 60 mg (1,6 mmol) NaH und rührte für 10 min bei RT. Nach Zugabe von 335 mg 2-(2-Bromomethyl-phenyl)-4,4,5,5-tetramethyl-[1,3,2]dioxaborolan wird bis zum max. Umsatz gerührt und mit MeOH gequencht. Man nimmt in ges. NaCl-Lösung/Ethylacetat auf, trocknet die organische Phase über Natriumsulfat, filtriert und entfernt das Lösungsmittel im Vakuum. Der Rückstand wird durch Flash-Chromatographie an Kieselgel (n-Heptan/Ethylacetat = 1:1) gereinigt. Man erhält (1S,3R)-5-Methyl-4-{3-[2-(4,4,5,5-tetramethyl-[1,3,2]dioxaborolan-2-yl)-benzyloxy]-cyclohexyloxymethyl}-2-m-tolyl-oxazol als farbloses Öl. C₃₁H₄₀BNO₅ (517.48), MS (ESI): 5-18 (M + H⁺).

### (1R,3S)-2-[3-(5-Methyl-2-m-tolyl-oxazol-4-ylmethoxy)-cyclohexyloxymethyl]-benzolboronsäure

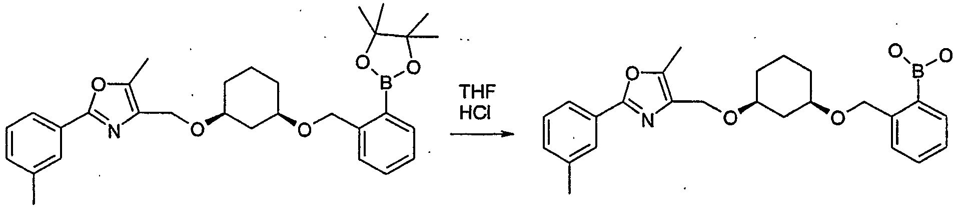

Zu 300 mg (1S,3R)-5-Methy)-4-{3-[2-(4,4,5,5-tetramethyl-[1,3,2]dioxaborolan-2-yl)-benzyloxy]-cyclohexyloxymethyl}-2-m-tolyl-oxazol in 5 ml THF gibt man 1 ml 1 N HCl und rührte bei RT bis zum vollständigen Umsatz. Man entfernt das Lösungsmittel im Vakuum und reinigt die Verbindung durch RP-HPLC. Man erhält (1 R,3S)-2-[3-(5-Methyl-2-m-tolyl-oxazol-4-ylmethoxy)-cyclohexyloxymethyl]-benzolboronsäure als farbloses Öl. C₂₅H₃₀BNO₅ (435.33), MS (ESI): 436. (M + H⁺).

### Beispiel XXVI

### 1-[(1S,3R)/(1R,3S)-3-(5-Methyl-2-p-tolyl-oxazol-4-ylmethoxy)-cyclohexylmethyl]-1H-indole-2-carbonsäure

### Cis-3-Hydroxymethylcyclohexanol

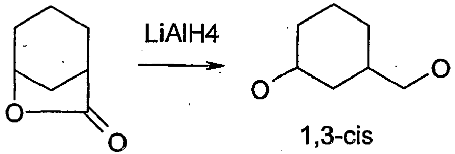

10 g 6-Oxa-bicyclo[3.2.1]octan-7-one werden in 300 ml Tetrahydrofuran gelöst und unter Eiskühlung mit 160 ml einer 1M Lösung von Lithiumaluminiumhydrid in Tewtrahydrofuran versetzt. Nach 30 Minuten Rühren bei Raumtemperatur wird gesättigte Ammoniumchloridlösung zugesetzt und durch Zugabe einer 5%igen Zitronensäurelösung ein neutraler pH-Wert eingestellt. Das Tetrahydrofuran wird im Vakuum entfernt und der Rückstand dreimal mit je 150 ml Ethylacetat extrahiert. Die vereinigten organischen Phasen werden über MgSO4 getrocknet und anschließend das Lösungsmittel im Vakuum entfernt. Man erhält 10.5 g (1 S,3R)/(1 R,3S)-3-Hydroxymethyl-cyclohexanol als farbloses Öl. C7H14O2 (130.13), Rf(Ethylacetat) = 0.14.

### (1S,3R)/(1R,3S)-3-(tert-Butyl-diphenyl-silanyloxymethyl)-cyclohexanol

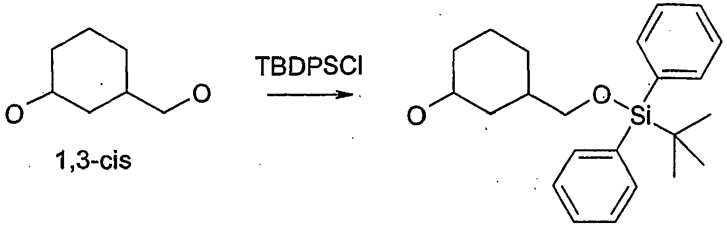

10.5 g (1 S,3R)/(1 R,3S)-3-Hydroxymethyl-cyclohexanol werden in 300 ml Dimethylformamid gelöst und mit 23 ml tert-Butyl-diphenyl-silanylchlorid, 8.0 g Imidazol und 200 mg Dimethylaminopyridin versetzt. Es wird 12 Stunden bei Raumtemperatur gerührt. Das Dimethylformamid wird im Vakuum entfernt, der Rückstand in 300 ml Ethylacetat gelöst und fünfmal mit je 100 ml Wasser gewaschen. Die organische Phase wird über MgSO4 getrocknet und anschließend das Lösungsmittel im Vakuum entfernt. Man erhält 27.0 g (1S,3R)/(1R,3S)-3-(tert-Butyl-diphenyl-silanyloxymethyl)-cyclohexanol als Öl. C23H32O2Si (368.6), Rf(n-Heptan:Ethylacetat = 1:1) = 0.42.

### 4-[(1S,3R)/(1R,3S)-3-(tert-Butyl-diphenyl-silanyloxymethyl)-cyclohexyloxymethyl]-5-methyl-2-p-tolyl- oxazol

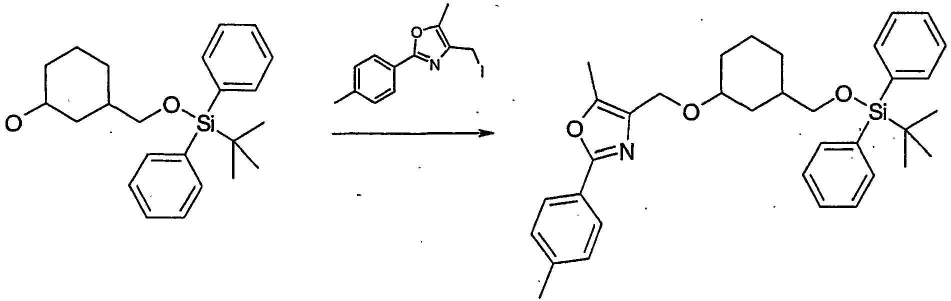

6.4 g (1 S,3R)/(1R,3S)-3-(tert-Butyl-diphenyl-silanyloxymethyl)-cyclohexanol werden mit 6.5 g 4-lodomethyl-5-methyl-2-p-tolyl-oxazole in 200 ml Dimethylformamid gelöst und mit 1 g Natriumhydrid (60%ige Suspension in Mineralöl) versetzt. Nach 1 Stunde Rühren bei Raumtemperatur werden nochmals 2 g Natriumhydrid und 5 g 4-lodomethyl-5-methyl-2-p-tolyl-oxazole zugegeben. Nach 4 stündigem Rühren bei Raumtemperatur wird das Reaktionsgemisch durch Zugabe von 400 ml Ethylacetat verdünnt und fünfmal mit je 200 ml Wasser gewaschen. Die organische Phase Phase wird über MgSO4 getrocknet und anschließend das Lösungsmittel im Vakuum entfernt. Der Rückstand wird an Kieselgel mit dem Eluens n-Heptan:Ethylacetat = 10:1 gereinigt. Man erhölt 6.8 g 4-[(1S,3R)/(1R,3S)-3-(tert-Butyl-diphenyl-silanyloxymethyl)-cyclohexyloxymethyl]-5-methyl-2-p-tolyl-oxazol als Öl. C35H43NO3Si (553.28), Rf(n-Heptan:Ethylacetat = 2:1) = 0.50.

### [(1S,3R)/(1R,3S)-3-(5-Methyl-2-p-tolyl-oxazol-4-ylmethoxy)-cyclohexyl]-methanol

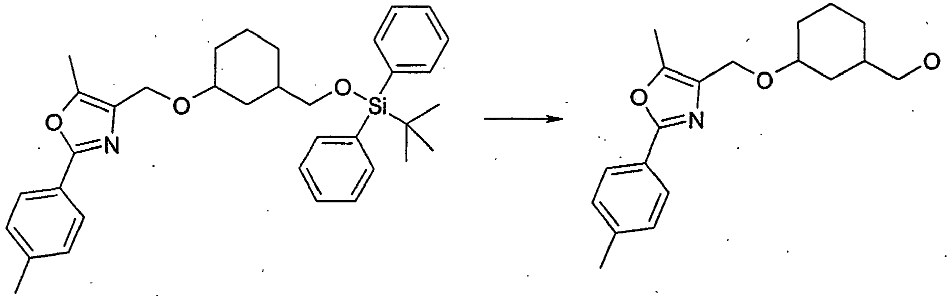

6.8 g 4-[(1S,3R)/(1R,3S)-3-(tert-Butyl-diphenyl-silanyloxymethyl)-cyclohexyloxymethyl]-5-methyl-2-p-tolyl-oxazol werden in 40 ml Tetrahydrofuran gelöst und mit 40 ml einer 1 M Lösung von Tetrabutylammoniumfluorid versetzt. Es wird 1 Stunde auf 50°C erwärmt, anschließend das Lösungsmittel im Vakuum entfernt und der resultierende Rückstand an Kieselgel mit dem Eluens n-Heptan:Ethylacetat = 5:1=> 1:1 gereinigt. Man erhält 1.0 g [(1S,3R)/(1R,3S)-3-(5-Methyl-2-p-tolyl-oxazol-4-ylmethoxy)-cyclohexyl]-methanol als Öl. C19H25NO3 (315.42), Rf(n-Heptan:Ethylacetat = 1:1) = 0.13.

### Toluene-4-sulfonsäure-(1 S,3R)/(1R,3S)-3-(5-methyl-2-p-tolyl-oxazol-4-ylmethoxy)-cyclohexylmethyl ester

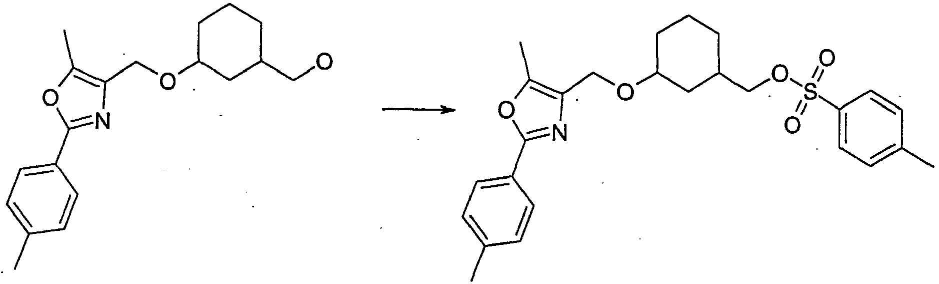

1 g [(1S,3R)/(1R,3S)-3-(5-Methyl-2-p-tolyl-oxazol-4-ylmethoxy)-cyclohexyl]-methanol werden zusammen mit 730 mg p-Toluolsulfonsäurechlorid, 0.7 ml Triethylamin und 50 mg Dimethylaminopyridin in 20 ml Dichlormethan gelöst und 24 h bei Raumtemperatur gerührt. Das Gemisch wird mit Wasser und gesättigter Natriumhydrogencarbonatlösung gewaschen, die organische Phase über MgSO4 getrocknet und anschließend das Lösungsmittel im Vakuum entfernt. Man erhält 1.5 g Toluene-4-sulfonsäure-(1S,3R)/(1 R,3S)- 3-(5-methyl-2-p-tolyl-oxazol-4-ylmethoxy)-cyclohexylmethylester als braunes Öl, das ohne Reinigung weiter umgesetzt wird. C26H31NO5S (469.60), Rf(n-Heptan:Ethylacetat = 1:1) = 0.50.

### 1-[(1 S,3R)/(1R,3S)-3-(5-Methyl-2-p-tolyl-oxazol-4-ylmethoxy)-cyclohexylmethyl]-1H-indole-2-carbonsäure

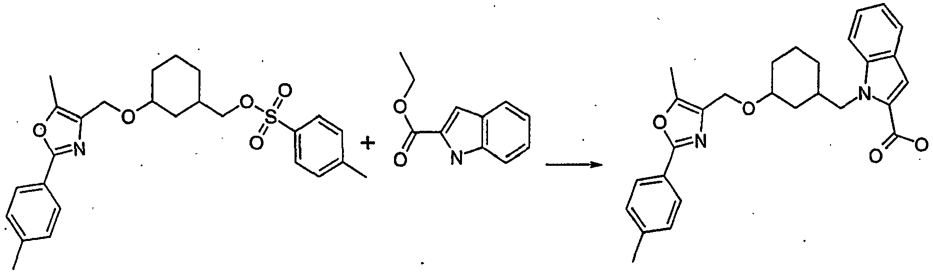

120 mg 1 H-Indole-2-carbonsäureethylester werden in 5ml Dimethylformamid gelöst und mit 25 mg Natriumhydrid (60%ige Suspension in Mineralöl) versetzt. Nach 30 Minuten werden 100 mg Toluene-4-sulfonsäure-(1S,3R)/(1R,3S)-3-(5-methyl-2-p-tolyl-oxazol-4-ylmethoxy)-cyclohexylmethylester, gelöst in 1 ml Dimethylformamid, zugetropft. Es werden nochmals 25 mg Natriumhydrid (60%ige Suspension in Mineralöl) hinzugefügt und das Reaktionsgemisch 3 Stunden auf 60°C erwärmt. Durch Zugabe von 50 ml Methyl-tert-butylether wird das Gemisch verdünnt und dreimal mit je 20 ml Wasser gewaschen. Die organische Phase wird über MgSO4 getrocknet und anschließend das Lösungsmittel im Vakuum entfernt. Der Rückstand wird mittels RP-HPLC gereinigt. Nach Gefriertrocknung erhält man 36 mg des Racemats 1-[(1S,3R)/(1R,3S)-3-(5-Methyl-2-p-tolyl-oxazol-4-ylmethoxy)-cyclohexyl-methyl]-1H-indole-2-carbonsäure als Lyophilisat. C28H30N2O4 (458.56), MS(ESI) = 459 (M+H⁺).

### Beispiel XXVII

### 1-[(1S,3R)/(1R,3S)-3-(5-Methyl-2-p-tolyl-oxazol-4-ylmethoxy)-cyclohexylmethyl]-1H-pyrrole-2- carbonsäure

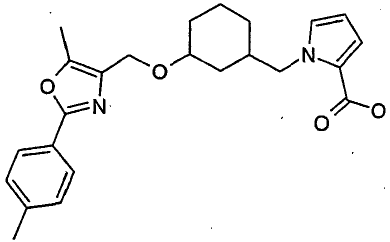

Analog zu **Beispiel XXVI** wurde aus Toluene-4-sulfonsäure-(1 S,3R)/(1 R,3S)-3-(5-methyl-2-p-tolyl-oxazol-4-ylmethoxy)-cyclohexylmethylester und 1H-Pyrrole-2-carbonsäureethylester das Racemat 1-[(1S,3R)/(1R,3S)-3-(5-Methyl-2-p-tolyl-oxazol-4-ylmethoxy)-cyclohexylmethyl]-1H-pyrrole-2- carbonsäure erhalten, C24H28N2O4 (408.50), MS(ESI) = 409 (M+H⁺).

### Beispiel XXVIII

### 1-{(1S,3R)/(1R,3S)-3-[2-(3-Methoxy-phenyl)-5-methyl-oxazol-4-ylmethoxy]-cyclohexylmethyl}-1H-pyrrole-2-carbonsäure

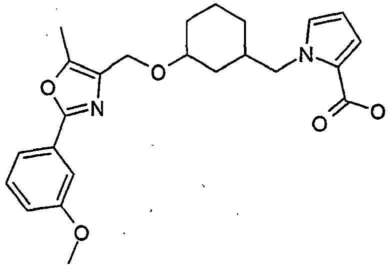

Analog zu **Beispiel XXVI** wurde aus 4-Iodomethyl-2-(3-methoxy-phenyl)-5-methyl-oxazole, (1S,3R)/(1R,3S)-3-(tert-Butyl-diphenyl-silanyloxymethyl)-cyclohexanol und 1 H-Pyrrole-2-carbonsäureethylester das Racemat 1-{(1 S,3R)/(1R,3S)-3-[2-(3-Methoxy-phenyl)-5-methyl-oxazol-4-ylmethoxy]-cyclohexylmethyl}-1H-pyrrole-2-carbonsäure erhalten, C24H28N2O5 (424.50), MS(ESI) = 425 (M+H⁺).

### Beispiel XXIX

### (1 S,3R)/(1R,3S)-3-(5-Methyl-2-p-tolyloxazol-4-ylmethoxy)-cyclohexylmethyloxy]-thiophen-2-carbonsäure:

### (1 S,3R)/(1R,3S)-3-(5-Methyl-2-p-tolyloxazol-4-ylmethoxy)-cyclohexylmethyloxy]-thiophen-2-carbonsäuremethytester:

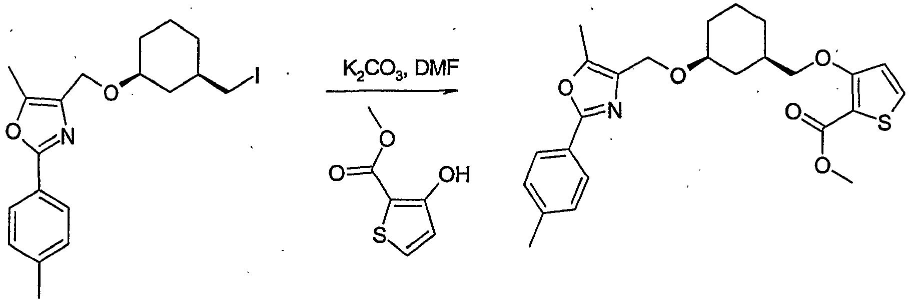

50 mg 4-((1S,3R)/(1R,3S)-3-lodmethylcyclohexyloxymethyl)-5-methyl-2-p-tolyl-oxazol in 2 ml DMF werden mit 27 mg 3-Hydroxythiophen-2-carbonsäuremethylester und 67 mg Kaliumcarbonat versetzt und 2 h bei 90 °C gerührt. Nach Zugabe von Wasser und MTBE werden die Phasen getrennt. Die organische Phase wird über MgSO4 getrocknet und eingeengt. Chromatographie des Rückstandes an Kieselgel (Heptan/Essigester 5/1) liefert 12 mg des Racemats (1S,3R)/(1R,3S)-3-(5-Methyl-2-p-tolyloxazol-4-ylmethoxy)-cyclohexylmethyloxy]-thiophen-2-carbonsäuremethylester als gelbes Öl. C25H29NO5S (455,58); LCMS (ESI): 456,1 (MH⁺).

### (1S,3R)/(1R,3S)-3-(5-Methyl-2-p-tolyloxazol-4-ylmethoxy)-cyclohexylmethyloxy]-thiophen-2-carbonsäure:

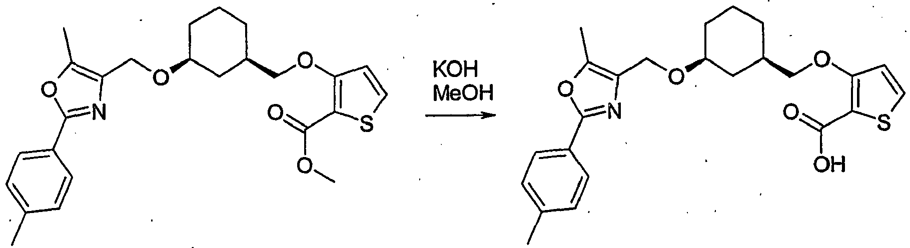

12 mg (1 S,3R)/(1R,3S)-3-(5-Methyl-2-p-tolyloxazol-4-ylmethoxy)-cyclohexylmethyloxy]-thiophen-2-carbonsäuremethylester 3-(5-Methyl-2-p-tolyloxazol-4-ylmethoxy)-cyclohexylmethyloxy]-thiophen-2-carbonsäuremethylester werden in 1 ml Methanol gelöst und mit 10 Tropfen 2N KOH versetzt und bei RT 1 h gerührt. Dann werden 2 ml gesättigte NH4Cl-Lösung und MTBE zugesetzt und die Phasen getrennt. Die organische Phase wird über MgSO4 getrocknet und eingeengt, wobei 9,4 mg (1S,3R)/(1R,3S)-3-(5-Methyl-2-p-tolyloxazol-4-ylmethoxy)-cyclohexylmethyloxy]-thiophen-2-carbonsäure als Racemat erhalten werden. C24H27NO5S (441,55); LCMS(ESI): 442,1 (MH⁺).

### Beispiel XXX

### (1 S,3R)/(1R,3S)-3-[3-(5-Methyl-2-p-tolyloxazol-4-ylmethoxy)-cyclohexylmethoxy]-5-trifluormethylthiophen-2-carbonsäure

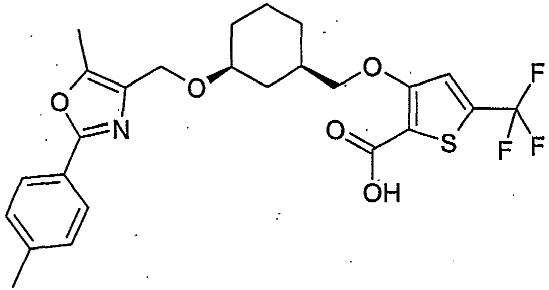

Analog zu **Beispiel XXIX** erhält man aus 4-((1 S,3R)/(1R,3S)-3-lodmethylcyclohexyloxymethyl)-5-methyl-2-p-tolyl-oxazol und 3-Hydroxy-5-trifluormethylthiophen-3-carbonsäuremethylester (1S,3R)/(1R,3S)-3-[3-(5-Methyl-2-p-tolyloxazol-4-ylmethoxy)-cyclohexylmethoxy]-5-trifluormethylthiophen-2-carbonsäure als Racemat. C25H26F3NO5S (509,55), LCMS(ESI): 510,1 (MH⁺).

### Beispiel XXXI

### 6-Chlor-3-[(1S,3R)/(1R,3S)-3-(5-methyl-2-p-tolyloxazol-4-ylmethoxy)-cyclohexylmethoxy]-benzo[b]thiophen-2-carbonsäure

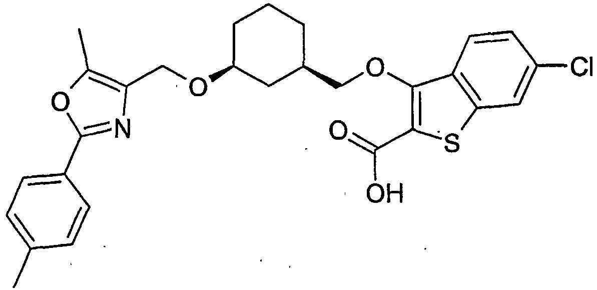

Analog zu **Beispiel XXIX** erhält man aus 4-((1S,3R)/(1 R,3S)-3-lodmethylcyclohexyloxymethyl)-5-methyl-2-p-tolyl-oxazol und 6-Chlor-3-hydroxybenzo[b]thiophen-2-carbonsäuremethylester 6-Chlor-3-[(1S,3R)/(1R,3S)-3-(5-methyl-2-p-tolyloxazol-4-ylmethoxy)-cyclohexylmethoxy]-benzo[b]thiophen-2-carbonsäure als Racemat. C28H28ClNO5S (526,06), LCMS(ESI): 526,1 (MH⁺).

## Patentansprüche

1. Verbindungen der Formel 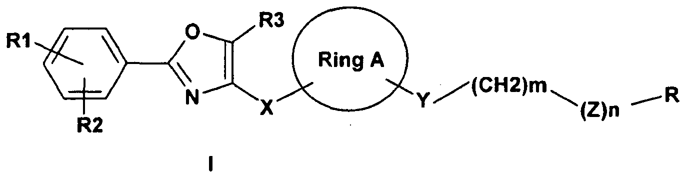 worin bedeuten:
Ring A Cyclohexan-1,3-diyl;
R1, R2 unabhängig voneinander H, F, Br, Cl, SF₅, S-(C₁-C₆)-Alkyl, CF₃, OCF₃, (C₁-C₆)-Alkyl, O-(Cᵢ-C₆)-Alkyl, SCF₃, Phenoxy, OCF₂CHF₂, OCF₂CF₃, (C₁-C₆)-Alkyl-(C₁-C₆)-Alkoxy, O(C₁-C₆)-Alkyl-(C₁-C₆)-Alkoxy, Benzyloxy;
R3 H, CF₃, (C₁-C₆)-Alkyl, (C₃-C₈)-Cycloalkyl, Phenyl;
X (C₁-C₆)-Alkandiyl, wobei in der Alkandiylgruppe ein oder mehrere Kohlenstoffatome durch Sauerstoffatome ersetzt sein können;
Y S, O, Bindung;
m 1 bis 3;
n 0 oder 1;
Z O, S, CO oder CO-NH;
R H, OH, CH₂-CO-NH-OH, CH₂-CO-NH-(C₁-C₆)-Alkyl, CH₂-CO-NH-(C₁-C₆)-Alkoxy, NR4R5 oder ein 5 - 12 gliedriger mono- oder bicyclischer, ungesättigter, teilweise ungesättigter oder gesättigter Ring, der ein bis vier Heteroatome aus der Gruppe N, O oder S enthalten und benzanneliert sein kann, wobei der 5-12 gliedrige Ring weitere Substituenten wie F,Cl, Br, CN, SH, COOH, (C₁C₄)-Alkyl, (C₁-C₆)-Alkoxy, SO₂-(C₁-C₄)-Alkyl, NO₂, CF₃, OCF₃, (C₁-C₆)-Alkyl-(C₁-C₆)-Alkoxy, (C₁-C₆)-Alkoxy-(C₁-C₆)-Alkoxy, (C₁-C₆)-Alkoxy-Phenyl, Phenoxy, NHSO₂CF₃ oder B(OH)2 tragen kann;
R4 H, (C₁-C₆)-Alkyl;
R5 OH, NH₂, SO₂-CF₃, SO₂-Phenyl-CF₃, CO-CF₃, (C₁-C₆)-Alkoxy, Phenyl, das gegebenenfalls substituiert sein kann durch CH₃ und COOH;
R4 und R5 zusammen mit dem sie tragenden N-Atom einen 5 gliedrigen aromatischen Heterocyclus bilden, der gegebenenfalls wiederum an einen aromatischen 5 - 7 gliedrigen Ring mit gegebenenfalls ein bis vier N-Atomen anneliert ist und substituiert sein kann durch: F, Cl, Br, CF₃, OCF₃, COOH, SO₂CH₃ CN, (C₁-C₄)-Alkoxy, (C₁-C₄)-Alkyl, (C₁-C₆)-Alkyl-Phenyl, (C₁-C₆)-Alkyl-(C₁-C₆)-Alkoxy, (C₁-C₆)-Alkoxy-(C₁-C₆)-Alkoxy, (C₁-C₆)-Alkoxy-Phenyl, Phenoxy;
sowie deren physiologisch verträgliche Salze,
wobei die Verbindungen der Formel I ausgenommen sind mit
Y und Z = O und R = Phenyl-COOH oder Phenyl-(C₁-C₄)-Alkyl und solche mit Y = O, n = 0 und R = Phenyl-COOH oder Phenyl-(C₁-C₄)-Alkyl.

2. Verbindungen der Formel I gemäß Anspruch 1, worin bedeuten
Ring A Cyclohexan-1,3-diyl, und
X (C₁-C₆)-Alkandiyl, worin ein Kohlenstoffatom durch ein Sauerstoffatom ersetzt sein kann.

3. Verbindungen der Formel I gemäß Anspruch 1 oder 2, worin bedeuten
Ring A Cyclohexan-1,3-diyl und
X CH₂-O.

4. Verbindungen der Formel I gemäß den Ansprüchen 1 bis 3, worin bedeuten
Ring A Cyclohexan-1,3-diyl;
X CH₂-O;
Y O.

5. Verbindungen der Formel I gemäß den Ansprüchen 1 bis 4, worin der zentrale Cycloalkan-1,3-diylring cis verknüpft ist

6. Verbindungen der Formel I gemäß den Ansprüchen 1 bis 5, worin bedeuten
R1/R2 H, (C₁-C₄)-Alkyl, (C₁-C₄)-Alkoxy;
R3 (C₁-C₄)-Alkyl.

7. Verbindungen der Formel I gemäß den Ansprüchen 1 bis 6, worin bedeuten
Y O;
m 3;
n 0.

8. Verbindungen der Formel I gemäß den Ansprüchen 1 bis 6, worin bedeuten
Y O;
m 2;
n 0.

9. Verbindungen der Formel I gemäß den Ansprüchen 1 bis 6, worin bedeuten
Y O;
m 2;
n 1;
Z O.

10. Verbindungen der Formel I gemäß den Ansprüchen 1 bis 6, worin bedeuten
Y O;
m 1;
n 0;

11. Verbindungen der Formel I gemäß den Ansprüchen 1, 2, 3, 5 und 6, worin bedeuten .
Y Bindung;
m 1;
n 0.

12. Verbindungen der Formel I gemäß den Ansprüchen 1, 2, 3, 5 und 6, worin bedeuten
Y Bindung;
m 1;
n 1;
Z O.

13. Verbindungen der Formel I gemäß den Ansprüchen 1 bis 7, worin bedeuten
Y O;
m 3;
n 0;
R Tetrazol, NHSO₂CF₃.

14. Verbindungen der Formel I gemäß den Ansprüchen 1 bis 6 und 8, worin bedeuten
Y O;
m 2;
n 0;
R Tetrazol, NHSO₂CF₃ oder NR4R5, das für Indol oder 6-Azaindol steht und substituiert sein kann durch F, Br, CN, COOH, (C₁C₄)-Alkyl, (C₁-C₄)-Alkoxy, SO₂-CH₃, (C₁-C₆)-Alkoxy-(C₁-C₆)-Alkoxy oder Benzoxy.

15. Verbindungen der Formel I gemäß den Ansprüchen 1 bis 6 und 9, worin bedeuten
Y O;
m 2;
n 1;
Z O;
R Phenyl oder Thiophen, die weitere Substituenten wie F, COOH, (C₁-C₄)-Alkyl, (C₁-C₄)-Alkoxy, NO₂,CF₃, Benzyloxy oder B(OH)₂ tragen können.

16. Verbindungen der Formel I gemäß den Ansprüchen 1 bis 6 und 10, worin bedeuten:
Y O;
m 1;
n 0;
R Phenyl-NHSO₂CF₃, Phenyl-B(OH)₂.

17. Verbindungen der Formel I gemäß den Ansprüchen 1, 2, 3, 5, 6 und 11, worin bedeuten
Y Bindung;
m 1;
n 0;
R NR4R5, das für Pyrrol oder Indol steht und durch COOH substituiert ist.

18. Verbindungen der Formel I gemäß den Ansprüchen 1, 2, 3, 5, 6 und 12, worin bedeuten
Y Bindung;
m 1;
n 1;
Z O;
R Thiophen oder Benzothiophen, die substituiert sein können durch COOH, Cl, CF₃.

19. Arzneimittel enthaltend eine oder mehrere der Verbindungen der Formel I gemäß einem oder mehreren der Ansprüche 1 bis 18.

20. Arzneimittel enthaltend eine oder mehrere der Verbindungen der Formel I gemäß einem oder mehreren der Ansprüche 1 bis 18 und einen oder mehrere Wirkstoffe, die günstige Wirkungen auf Stoffwechselstörungen oder damit assozierte Erkrankungen haben.

21. Arzneimittel enthaltend eine oder mehrere der Verbindungen der Formel I gemäß einem oder mehreren der Ansprüche 1 bis 18 und einen oder mehrere Antidiabetika.

22. Arzneimittel enthaltend eine oder mehrere der Verbindungen der Formel I gemäß einem oder mehreren der Ansprüche 1 bis 18 und einen oder mehrere Lipidmodulatoren.

23. Verbindungen der Formel I gemäß einem oder mehreren der Ansprüche 1 bis 18 zur Herstellung eines Arzneimittels zur Behandlung und/oder Prävention von Störungen des Fettsäurestoffwechsels und Glucoseverwertungsstörungen.

24. Verbindungen der Formel I gemäß einem oder mehreren der Ansprüche 1 bis 18 zur Herstellung eines Arzneimittels zur Behandlung und/oder Prävention von Störungen, bei denen Insulin Resistenz eine Rolle spielt.

25. Verwendung der Verbindungen der Formel I gemäß einem oder mehreren der Ansprüche 1 bis 18 zur Herstellung eines Arzneimittels zur Behandlung und/oder Prävention von Diabetes mellitus und der damit verbundenen Folgeerkrankungen.

26. Verbindungen der Formel I gemäß einem oder mehreren der Ansprüche 1 bis 18 zur Herstellung eines Arzneimittels zur Behandlung und/oder Prävention von Dyslipidämien und deren Folgen.

27. Verbindungen der Formel I gemäß einem oder mehreren der Ansprüche 1 bis 18 zur Herstellung eines Arzneimittels zur Behandlung und/oder Prävention von Zuständen, die mit dem Metabolischen Syndrom assoziert sind.

28. Verbindungen gemäß einem oder mehreren der Ansprüche 1 bis 18 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

29. Verbindungen gemäß einem oder mehreren der Ansprüche 1 bis 18 in Kombination mit mindestens einem weiteren Wirkstoff zur Herstellung eines Arzneimittels zur Behandlung und/oder Prävention von Störungen, bei denen Insulin Resistenz eine Rolle spielt.

30. Verfahren zur Herstellung eines Arzneimittels enthaltend eine oder mehrere der Verbindungen gemäß einem oder mehreren der Ansprüche 1 bis 18, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 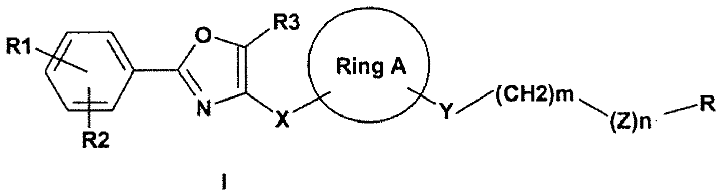 in which
ring A is cyclohexane-1,3-diyl;
R1, R2 independently of one another are H, F, Br, Cl, SF₅, S-(C₁-C₆)-alkyl, CF₃, OCF₃, (C₁-C₆)-alkyl, O-(C₁-C₆)-alkyl, SCF₃, phenoxy, OCF₂CHF₂, OCF₂CF₃, (C₁-C₆-alkyl-(C₁-C₆)-alkoxy, O(C₁-C₆)-alkyl-(C₁-C₆)-alkoxy, benzyloxy;
R3 is H, CF₃, (C₁-C₆)-alkyl, (C₃-C₈)-cycloalkyl, phenyl;
X is (C₁-C₆)-alkanediyl, where in the alkanediyl group one or more carbon atoms may be replaced by oxygen atoms;
Y is S, 0, a bond;
m is 1 to 3;
n is 0 or 1;
Z is 0, S, CO or CO-NH;
R is H, OH, CH₂-CO-NH-OH, CH₂-CO-NH-(C₁-C₆)-alkyl, CH₂-CO-NH-(C₁-C₆)-alkoxy, NR4R5 or a 5- to 12-membered mono or bicyclic unsaturated, partially unsaturated or saturated ring which may contain one to four heteroatoms from the group consisting of N, O. and S and which may be benzo-fused, where the 5- to 12-membered ring may carry further substituents such as F, Cl, Br, CN, SH, COOH, (C₁-C₄)-alkyl, (C₁-C₆)-alkoxy, SO₂-(C₁-C₄)-alkyl, NO₂,CF₃, OCF₃, (C₁-C₆)-alkyl-(C₁-C₆)-alkoxy, (C₁-C₆)-alkoxy-(C₁-C₆)-alkoxy, (C₁-C₆) -alkoxy-phenyl, phenoxy NHSO₂CF₃ or B(OH)₂;
R4 is H, (C₁-C₆)-alkyl;
R5 is OH, NH₂, SO₂-CF₃, SO₂-phenyl-CF₃, CO-CF₃, (C₁-C₆)-alkoxy, phenyl which may be substituted by CH₃ and COOH;
R4 and R5 together with the nitrogen atom that carries them form a 5-membered aromatic heterocycle which in turn may be fused to an aromatic 5- to 7-membered ring which may have one to four nitrogen atoms and which may be substituted by: F, Cl, Br, CF₃, OCF₃, COOH, SO₂CH₃ CN, (C₁-C₄)-alkoxy, (C₁-C₉) -alkyl, (C₁-C₆)-alkyl-phenyl, (C₁-C₆)-alkyl-(C₁-C₆)-alkoxy, (C₁-C₆)-alkoxy-(C₁-C₆) -alkoxy, (C₁-C₆)-alkoxy-phenyl, phenoxy;
and physiologically acceptable salts thereof,
the compounds of the formula I being excluded where Y and Z = 0 and R = phenyl-COOH or phenyl-(C₁-C₄)-alkyl and those where Y = O, n = 0 and R = phenyl-COOH or phenyl-(C₁-C₄)-alkyl.

2. A compound of the formula I as claimed in claim 1, in which
ring A is cyclohexane-1,3-diyl, and
X is (C₁-C₆)-alkanediyl, where one carbon atom may be replaced by an oxygen atom.

3. A compound of the formula I as claimed in claim 1 or 2, in which
ring A is cyclohexane-1,3-diyl and
X is CH₂-O.

4. A compound of the formula I as claimed in any of claims 1 to 3, in which
ring A is cyclohexane-1,3-diyl;
X is CH₂-O;
Y is O.

5. A compound of the formula I as claimed in any of claims 1 to 4, in which the central cycloalkane-1,3-diyl ring is attached cis.

6. A compound of the formula I as claimed in any of claims 1 to 5, in which
R1/R2 are H, (C₁-C₄)-alkyl, (C₁-C₄)-alkoxy;
R3 is (C₁-C₄)-alkyl.

7. A compound of the formula I as claimed in any of claims 1 to 6, in which
Y is 0;
m is 3;
n is 0.

8. A compound of the formula I as claimed in any of claims 1 to 6, in which
Y is 0;
m is 2;
n is 0.

9. A compound of the formula I as claimed in any of claims 1 to 6, in which
Y is 0;
m is 2;
n is 1;
Z is O.

10. A compound of the formula I as claimed in any of claims 1 to 6, in which
Y is 0;
m is 1;
n is 0;

11. A compound of the formula I as claimed in any of claims 1, 2, 3, 5 and 6, in which
Y is a bond;
m is 1;
n is 0.

12. A compound of the formula I as claimed in any of claims 1, 2, 3, 5 and 6, in which
Y is a bond;
m is 1;
n is 1;
Z is O.

13. A compound of the formula I as claimed in any of claims 1 to 7, in which
Y is O;
m is 3;
n is 0;
R is tetrazole, NHSO₂CF₃.

14. A compound of the formula I as claimed in any of claims 1 to 6 and 8, in which
Y is 0;
m is 2;
n is 0;
R is tetrazole, NHSO₂CF₃ or NR4R5 which denotes indole or 6-azaindole and may be substituted by F, Br, CN, COOH, (C₁C₄)-alkyl, (C₁-C₄)-alkoxy, SO₂-CH₃, (C₁-C₆)-alkoxy-(C₁-C₆)-alkoxy or benzoxy.

15. A compound of the formula I as claimed in any of claims 1 to 6 and 9, in which
Y is 0;
m is 2;
n is 1;
Z is 0;
R is phenyl or thiophene, which radicals may carry further substituents such as F, COOH, (C₁-C₄)-alkyl, (C₁-C₄)-alkoxy, NO₂,CF₃, benzyloxy or B(OH)₂.

16. A compound of the formula I as claimed in any of claims 1 to 6 and 10, in which
Y is O;
m is 1;
n is 0;
R is phenyl-NHSO₂CF₃, phenyl-B(OH)₂.

17. A compound of the formula I as claimed in any of claims 1, 2, 3, 5, 6 and 11, in which
Y is a bond;
m is 1;
n is 0;
R is NR4R5 which denotes pyrrole or indole and is substituted by COOH.

18. A compound of the formula I as claimed in any of claims 1, 2, 3, 5, 6 and 12, in which
Y is a bond;
m is 1;
n is 1;
Z is 0;
R is thiophene or benzothiophene, both which may be substituted by COOH, Cl, CF₃.

19. A pharmaceutical comprising one or more of the compounds of the formula I as claimed in one or more of claims 1 to 18.

20. A pharmaceutical comprising one or more of the compounds of the formula I as claimed in one or more of claims 1 to 18 and one or more active compounds which act favorably on metabolic disorders or associated sequelae.

21. A pharmaceutical, comprising one or more compounds of the formula I as claimed in one or more of claims 1 to 18 and one or more antidiabetics.

22. A pharmaceutical comprising one or more of the compounds of the formula I as claimed in one or more of claims 1 to 18 and one or more lipid modulators.

23. A compound of the formula I as claimed in one or more of claims 1 to 18 for preparing a pharmaceutical for the treatment and/or prevention of disorders of the fatty acid metabolism and disorders of glycose utilization.

24. A compound of the formula I as claimed in one or more of claims 1 to 18 for preparing a pharmaceutical for the treatment and/or prevention of disorders in which insulin resistance plays a role.

25. A compound of the formula I as claimed in one or more of claims 1 to 18 for preparing a pharmaceutical for the treatment and/or prevention of diabetes mellitus and the associated sequelae.

26. A compound of the formula I as claimed in one or more of claims 1 to 18 for preparing a pharmaceutical for the treatment and/or prevention of dyslipidemias and their sequelae.

27. A compound of the formula I as claimed in one or more of claims 1 to 18 for preparing a pharmaceutical for the treatment and/or prevention of conditions associated with metabolic syndrome.

28. A compound as claimed in one or more of claims 1 to 18 in combination with at least one further active compound for preparing a pharmaceutical for the treatment and/or prevention of disorders of the fatty acid metabolism and disorders of glucose utilization.

29. A compound as claimed in one or more of claims 1 to 18 in combination with at least one further active compound for preparing a pharmaceutical for the treatment and/or prevention of disorders in which insulin resistance plays a role.

30. A process for preparing a pharmaceutical comprising one or more of the compounds as claimed in one or more of claims 1 to 18, which comprises mixing the active compound with a pharmaceutically suitable carrier and bringing this mixture into a form suitable for administration.

## Revendications

1. Composés de formule I 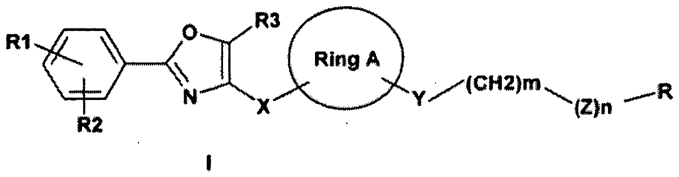 dans laquelle :
Ring A signifie cyclohexane-1,3-diyle ;
R1, R2 signifient indépendamment l'un de l'autre H, F, Br, Cl, SF₅, S-alkyle en (C₁-C₆), CF₃, OCF₃, alkyle en (C₁-C₆), O-alkyle en (C₁-C₆), SCF₃, phénoxy, OCF₂CHF₂, OCF₂CF₃, alkyle en (C₁-C₆)-alcoxy en (C₁-C₆), O-alkyle en (C₁-C₆)-alcoxy en (C₁-C₆), benzyloxy ;
R3 signifie H, CF₃, alkyle en (C₁-C₆), cycloalkyle en (C₃-C₈), phényle;
X signifie alcanediyle en (C₁-C₆), un ou plusieurs atomes de carbone dans le groupe alcanediyle pouvant être remplacés par des atomes d'oxygène ;
Y signifie S, 0, liaison ;
m signifie 1 à 3 ;
n signifie 0 ou 1 ;
Z signifie 0, S, CO ou CO-NH ;
R signifie H, OH, CH₂-CO-NH-OH, CH₂-CO-NH-alkyle en (C₁-C₆), CH₂-CO-NH-alcoxy en (C₁-C₆), NR4R5 ou un cycle de 5 à 12 éléments, mono- ou bicyclique, insaturé, partiellement insaturé ou saturé, qui peut contenir un à quatre hétéroatomes du groupe N, 0 ou S et peut être benzannelé, le cycle de 5 à 12 éléments pouvant porter des substituants supplémentaires tels que F, Cl, Br, CN, SH, COOH, alkyle en (C₁-C₉), alcoxy en (C₁-C₆), SO₂-alkyle en (C₁-C₄), NO₂,CF₃, OCF₃, alkyle en (C₁-C₆)-alcoxy en (C₁-C₆), alcoxy en (C₁-C₆)-alcoxy en (C₁-C₆), alcoxy en (C₁-C₆)-phényle, phénoxy, NHSO₂CF₃ ou B(OH)₂ ;
R4 signifie H, alkyle en (C₁-C₆) ;
R5 signifie OH, NH₂, SO₂-CF₃, SO₂-phényle-CF₃, CO-CF₃, alcoxy en (C₁-C₆), phényle, qui peut éventuellement être substitué par CH₃ et COOH ;
R4 et R5 forment ensemble avec l'atome N les portant un hétérocycle aromatique à 5 éléments, qui est éventuellement annelé à son tour à un cycle aromatique de 5 à 7 éléments contenant éventuellement un à quatre atomes N et peut être substitué par : F, Cl, Br, CF₃, OCF₃, COOH, SO₂CH₃ CN, alcoxy en (C₁-C₄), alkyle en (C₁-C₄), alkyle en (C₁-C₆)-phényle, alkyle en (C₁-C₆)-alcoxy en (C₁-C₆), alcoxy en (C₁-C₆)-alcoxy en (C₁-C₆), alcoxy en (C₁-C₆)-phényle, phénoxy ;
ainsi que leurs sels physiologiquement acceptables ;
les composés de formule I dans lesquels Y et Z = 0 et R = phényl-COOH ou phényl-alkyle en (C₁-C₄), et ceux dans lesquels Y = 0, n = 0 et R = phényl-COOH ou phényl-alkyle en (C₁-C₄) étant exclus.

2. Composés de formule I selon la revendication 1, dans lesquels
Ring A signifie cyclohexane-1,3-diyle et
X signifie alcanediyle en (C₁-C₆), dans lequel un atome de carbone peut être remplacé par un atome d'oxygène.

3. Composés de formule I selon la revendication 1 ou 2, dans lesquels
Ring A signifie cyclohexane-1,3-diyle et
X signifie CH₂-O.

4. Composés de formule I selon les revendications 1 à 3, dans lesquels
Ring A signifie cyclohexane-1,3-diyle ;
X signifie CH₂-O ;
Y signifie O.

5. Composés de formule I selon les revendications 1 à 4, dans lesquels le cycle cycloalcane-1,3-diyle central est relié en cis.

6. Composés de formule I selon les revendications 1 à 5, dans lesquels
R1/R2 signifient H, alkyle en (C₁-C₄), alcoxy en (C₁-C₄);
R3 signifie alkyle en (C₁-C₄).

7. Composés de formule I selon les revendications 1 à 6, dans lesquels
Y signifie 0 ;
m signifie 3 ;
n signifie 0.

8. Composés de formule I selon les revendications 1 à 6, dans lesquels
Y signifie 0 ;
m signifie 2 ;
n signifie 0.

9. Composés de formule I selon les revendications 1 à 6, dans lesquels
Y signifie 0 ;
m signifie 2 ;
n signifie 1 ;
Z signifie O.

10. Composés de formule I selon les revendications 1 à 6, dans lesquels
Y signifie O ;
m signifie 1 ;
n signifie 0.

11. Composés de formule I selon les revendications 1, 2, 3, 5 et 6, dans lesquels
Y signifie une liaison ;
m signifie 1 ;
n signifie 0.

12. Composés de formule I selon les revendications 1, 2, 3, 5 et 6, dans lesquels
Y signifie une liaison ;
m signifie 1 ;
n signifie 1 ;
Z signifie O.

13. Composés de formule I selon les revendications 1 à 7, dans lesquels
Y signifie O ;
m signifie 3 ;
n signifie 0 ;
R signifie tétrazole, NHSO₂CF₃.

14. Composés de formule I selon les revendications 1 à 6 et 8, dans lesquels
Y signifie O ;
m signifie 2 ;
n signifie 0 ;
R signifie tétrazole, NHSO₂CF₃ ou NR4R5, qui représente indole ou 6-azaindole et peut être substitué par F, Br, CN, COOH, alkyle en (C₁-C₄), alcoxy en (C₁-C₄), SO₂-CH₃, alcoxy en (C₁-C₆)-alcoxy en (C₁-C₆) ou benzoxy.

15. Composés de formule I selon les revendications 1 à 6 et 9, dans lesquels
Y signifie O ;
m signifie 2 ;
n signifie 1 ;
Z signifie 0 ;
R signifie phényle ou thiophène, qui peuvent porter des substituants supplémentaires tels que F, COOH, alkyle en (C₁-C₄), alcoxy en (C₁-C₄), NO₂, CF₃, benzyloxy ou B(OH)₂.

16. Composés de formule I selon les revendications 1 à 6 et 10, dans lesquels
Y signifie O ;
m signifie 1 ;
n signifie 0 ;
R signifie phényl-NHSO₂CF₃, phényl-B(OH)₂.

17. Composés de formule I selon les revendications 1, 2, 3, 5, 6 et 11, dans lesquels
Y signifie une liaison ;
m signifie 1 ;
n signifie 0 ;
R signifie NR4R5, qui représente pyrrole ou indole et est substitué par COOH.

18. Composés de formule I selon les revendications 1, 2, 3, 5, 6 et 12, dans lesquels
Y signifie une liaison ;
m signifie 1 ;
n signifie 1 ;
Z signifie O ;
R signifie thiophène ou benzothiophène, qui peuvent être substitués par COOH, Cl, CF₃.

19. Médicament contenant un ou plusieurs des composés de formule I selon une ou plusieurs des revendications 1 à 18.

20. Médicament contenant un ou plusieurs des composés de formule I selon une ou plusieurs des revendications 1 à 18 et un ou plusieurs agents actifs présentant des effets avantageux sur les troubles du métabolisme ou les maladies associées.

21. Médicament contenant un ou plusieurs des composés de formule I selon une ou plusieurs des revendications 1 à 18 et un ou plusieurs antidiabétiques.

22. Médicament contenant un ou plusieurs des composés de formule I selon une ou plusieurs des revendications 1 à 18 et un ou plusieurs modulateurs de lipides.

23. Composés de formule I selon une ou plusieurs des revendications 1 à 18 pour la fabrication d'un médicament pour le traitement et/ou la prévention de troubles du métabolisme des acides gras et de troubles de l'utilisation du glucose.

24. Composés de formule I selon une ou plusieurs des revendications 1 à 18 pour la fabrication d'un médicament pour le traitement et/ou la prévention de troubles dans lesquels la résistance à l'insuline joue un rôle.

25. Utilisation des composés de formule I selon une ou plusieurs des revendications 1 à 18 pour la fabrication d'un médicament pour le traitement et/ou la prévention du diabète sucré et des complications associées.

26. Composés de formule I selon une ou plusieurs des revendications 1 à 18 pour la fabrication d'un médicament pour le traitement et/ou la prévention des dyslipidémies et leurs conséquences.

27. Composés de formule I selon une ou plusieurs des revendications 1 à 18 pour la fabrication d'un médicament pour le traitement et/ou la prévention des états associés avec le syndrome métabolique.

28. Composés selon une ou plusieurs des revendications 1 à 18 en combinaison avec au moins un autre agent actif pour la fabrication d'un médicament pour le traitement et/ou la prévention de troubles du métabolisme des acides gras et de troubles de l'utilisation du glucose.

29. Composés selon une ou plusieurs des revendications 1 à 18 en combinaison avec au moins un autre agent actif pour la fabrication d'un médicament pour le traitement et/ou la prévention de troubles dans lesquels la résistance à l'insuline joue un rôle.

30. Procédé de fabrication d'un médicament contenant un ou plusieurs des composés selon une ou plusieurs des revendications 1 à 18, **caractérisé en ce que** l'agent actif est mélangé avec un véhicule pharmaceutiquement approprié et ce mélange est mis sous une forme appropriée pour l'administration.
